(19) Europäisches Patentamt
European Patent Office
Office européen des brevets

(11) EP 1 434 567 B1

(12) FASCICULE DE BREVET EUROPEEN

(45) Date de publication et mention de la délivrance du brevet:
**19.12.2007 Bulletin 2007/51**

(21) Numéro de dépôt: **02796803.1**

(22) Date de dépôt: **11.10.2002**

(51) Int Cl.:
***A61K 9/16*** *(2006.01)* ***A61K 47/48*** *(2006.01)*

(86) Numéro de dépôt international:
**PCT/FR2002/003474**

(87) Numéro de publication internationale:
**WO 2003/043604 (30.05.2003 Gazette 2003/22)**

(54) **PROCEDE DE PREPARATION D'UN COMPOSE D'INTERACTION DE SUBSTANCES ACTIVES AVEC UN SUPPORT POREUX PAR FLUIDE SUPERCRITIQUE**

HERSTELLUNGSVERFAHREN EINER INTERAKTIONVERBINDUNG VON WIRKSTOFFEN MIT EINEM PORÖSEN TRÄGER DURCH EIN ÜBERKRITISCHES FLUIDUM

METHOD FOR PREPARING A COMPOUND FOR INTERACTION OF ACTIVE SUBSTANCES WITH A POROUS SUPPORT USING SUPERCRITICAL FLUID

(84) Etats contractants désignés:
**AT BE BG CH CY CZ DE DK EE ES FI FR GB GR IE IT LI LU MC NL PT SE SK TR**

(30) Priorité: **12.10.2001 FR 0113178**

(43) Date de publication de la demande:
**07.07.2004 Bulletin 2004/28**

(73) Titulaire: **PIERRE FABRE MEDICAMENT
92100 Boulogne-Billancourt (FR)**

(72) Inventeurs:
- **FREISS, Bernard
F-81100 Castres (FR)**
- **MARCIACQ, Florence
F-13660 Orgon (FR)**
- **FAGES, Jacques
F-81000 Albi (FR)**
- **SAUCEAU, Martial
F-81000 Albi (FR)**
- **LOCHARD, Hubert
F-81000 Albi (FR)**
- **LETOURNEAU, Jean-Jacques
F-31380 Gragnague (FR)**
- **JOUSSOT-DUBIEN, Christophe
F-30650 Rochefort Du Gard (FR)**

(74) Mandataire: **Callon de Lamarck, Jean-Robert et al
Cabinet Régimbeau
20, rue de Chazelles
75847 Paris cedex 17 (FR)**

(56) Documents cités:
**WO-A-02/32462 WO-A-89/09639**

- **PATENT ABSTRACTS OF JAPAN vol. 012, no. 398 (C-538), 21 octobre 1988 (1988-10-21) & JP 63 141559 A (KINJIRUSHI WASABI KK;OTHERS: 01), 14 juin 1988 (1988-06-14)**
- **DATABASE CA [en ligne] CHEMICAL ABSTRACTS SERVICE, COLUMBUS, OHIO, US; VAN HEES, THIERRY ET AL: "Application of supercritical carbon dioxide for the preparation of a piroxicam-.beta.-cyclodextrin inclusion compound" retrieved from STN Database accession no. 132:185362 CA XP002203239 cité dans la demande & PHARMACEUTICAL RESEARCH (1999), 16(12), 1864-1870, 1999,**
- **DATABASE CA [en ligne] CHEMICAL ABSTRACTS SERVICE, COLUMBUS, OHIO, US; CHOI, YOUNG HAE ET AL: "Supercritical carbon dioxide extraction of Podophyllotoxin from Dysosma pleianthum roots" retrieved from STN Database accession no. 129:38803 CA XP002203240 & PLANTA MEDICA (1998), 64(5), 482-483, 1998,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 434 567 B1

## Description

La présente invention concerne un procédé d'interaction de substance active nanoparticulaire avec un support poreux, par la technologie des fluides supercritiques, en particulier celle du $CO_2$.

Les nouvelles molécules pharmaceutiques, à forte valeur ajoutée, sont dans 40% des cas insolubles ou peu solubles dans l'eau, ce qui nuit à leur biodisponibilité. L'augmentation de la surface spécifique des poudres permet d'améliorer leur vitesse de dissolution.

Or la biodisponibilité de principes actifs peut être considérablement augmentée si leur vitesse de dissolution est améliorée.

La génération de poudres fines de surfaces spécifiques élevées par la technologie des fluides supercritiques est utilisée depuis une quinzaine d'année.

Deux types de procédés sont classiquement mis en oeuvre : le procédé RESS (Rapid Expansion of Supercritical Solution), et le procédé SAS (Solvant-Anti-Solvant). Par modification des conditions opératoires, il est possible de contrôler la morphologie et la taille des particules formées de substance active.

Les avantages d'utilisation du $CO_2$ supercritique en tant que solvant sont multiples :

- Possibilité de travailler à basse température (>31°C) pour les substances actives thermosensibles,
- Pouvoir solvant facilement modulable en jouant sur les paramètres du procédé (pression, température, débit...),
- Séparation facile du mélange solvant-soluté par simple décompression,
- Inertie chimique du solvant : non-toxique, non-inflammable, non-corrosif,
- Coût moindre en comparaison des solvants organiques classiquement utilisés.

Dans les domaines pharmaceutique, cosmétique et nutraceutique, il existe un certain nombre de brevets et publications relatifs à la microencapsulation d'une substance active dans un agent enrobant. Néanmoins, la plupart des procédés décrits ne concernent pas l'amélioration de la biodisponibilité, mais plutôt l'adsorption d'une substance active sur un support.

Bertucco et al. (Drugs encapsulation using a compressed gas antisolvent technique - Proceedings of the 4th Italian Conference on Supercitical Fluids and their Applications 1997, 327-334 - Ed. E. Reverch*on*) décrivent un procédé dans lequel, on met en suspension la substance active dans une solution de bio polymère jouant le rôle du support. Cette suspension, placée dans l'autoclave, est ensuite mise en présence de $CO_2$ supercritique pour la désolvater (extraction du solvant par fluide supercritique) et entraîner la complexation du support par sursaturation sur la substance active. Ce procédé est un procédé batch, dans lequel la substance active n'est pas précipitée par le fluide supercritique puisqu'elle est en suspension. La structure des particules de substance active est donc inchangée, ce qui ne contribue pas à améliorer sa dissolution dans un milieu aqueux.

Un procédé identique est décrit par Benoît et al. dans leur demande de brevet WO98/13136.

Une autre technique de déposition d'un support, consiste à solubiliser ledit support dans le fluide supercritique, puis à faire précipiter ce support sur la substance active. Pour ce faire, la substance active et son support sont préalablement placés dans l'autoclave agité, et l'injection de $CO_2$ supercritique solubilise uniquement le support (ceci implique que le support soit soluble dans le fluide supercritique et que la substance active ne le soit pas), qui est précipité par modification de la pression et de la température au sein de l'autoclave. Dans ce cas, la structure initiale de la substance active reste inchangée, et il est difficile de maîtriser le ratio substance active/support obtenu dans le complexe précipité. Ce procédé batch est détaillé dans la demande de brevet EP 706 821 de Benoît et al.

Le procédé de microencapsulation décrit par Shine et Gelb dans leur demande de brevet WO98/15348 consiste en :

1. Mélanger une substance active avec un polymère d'encapsulation,
2. Liquéfier le polymère par passage d'un flux de fluide supercritique,
3. Dépressuriser rapidement de façon à solidifier le polymère autour de la substance active.

Ce procédé n'est applicable qu'avec une substance active et un polymère insolubles dans le fluide supercritique. De ce fait, la substance active conserve sa structure d'origine, ce qui ne contribue pas à améliorer sa biodisponibilité.

Dans la demande de brevet FR2798863 de Perrut et Majewski, la substance active (kava-kava, curcuma, mélange de poivre noir et de paprika doux), préalablement extraite par fluide supercritique, est précipitée dans un autoclave contenant un support poreux. Le milieu poreux étudié est la maltodextrine. Il s'agit donc d'une simple inclusion dans un support poreux sans étape de diffusion en mode statique de la substance active dans son support. Or la précipitation sur un support n'est pas suffisante pour améliorer de façon conséquente la solubilité de la substance active en milieu aqueux.

L'équipe de Tomasko (Chou et al., GAS crystallization of polymer-pharmaceutical composite particles, Proceedings of the 4'th International Symposium on Supercritical Fluids, 1997, 55-57 and Kim J.-H. et al., Microencapsulation of Naproxen using Rapid Expansion of Supercritical Solutions, Biotechnol. Prog. 1996, 12, 650-661) mentionne deux procédés de co-précipitation par RESS et par SAS avec du $CO_2$ supercritique. La substance active étudiée est le naproxène, tandis que le support est l'acide poly-L-lactique (L-PLA). Ces deux composés sont dissous simultanément dans l'acétone avant d'être précipités par injection de $CO_2$ à contre-courant, dans le cas du procédé SAS. Le complexe ainsi formé est récupéré après un temps de lavage. Un mélange de naproxène et de L-PLA est placé dans une enceinte, à partir de laquelle les deux composés sont extraits par le fluide supercritique, et précipités dans un second autoclave, pour ce qui concerne le procédé RESS. Or la précipitation ou co-précipitation d'une substance active et d'un support n'est pas suffisante pour améliorer de façon conséquente la solubilité de la substance active en milieu aqueux . De plus, là encore, aucune étape de diffusion moléculaire en mode statique afin d'améliorer l'interpénétration de la substance active avec son support n'est décrite dans ces deux procédés. Enfin la solubilité de la substance active dans un milieu aqueux n'est pas étudiée.

Il en est de même pour les procédés de co-précipitation décrits par Sze Tu et al. (Applications of dense gases in pharmaceutical processing, Proceedings of the 5th Meeting on Supercritical Fluids 1998, Tome 1, 263-269), Weber et al. (Coprecipitation with compressed antisolvents for the manufacture of microcomposites, Proceedings of the 5th Meeting on Supercritical Fluids 1998, Tome 1, 243-248) et Bleich et Müller (Production of drug loaded by the use of supercritical gases with the Aerosol Solvent Extraction System (ASES) process, J. Microencapsulation 1996, 13, 131-139).

Subramaniam et al. dans leur demande de brevet WO97/31691 ont développé un équipement et un procédé à partir d'antisolvants proches du point critique et supercritiques, qui permet de précipiter et d'enrober des particules. La phase de contact entre la solution, la suspension contenant le soluté, et l'antisolvant supercritique se fait de telle sorte qu'elle génère des ondes de hautes fréquences, qui divisent la solution en une multitude de petites gouttes. Dans ce brevet, la taille des particules revendiquée est de 0,1 à 10 $\mu$m. Par ailleurs, des procédés d'enrobage sont également décrits. Les cristallisations de l'hydrocortisone, du poly(D,L-lactide-glycolidique), de l'ibuprofène et de la camptothécine sont décrites. Or la précipitation ou co-précipitation d'une substance active et d'un support n'est pas suffisante pour améliorer de façon conséquente la solubilité de la substance active en milieu aqueux. En outre, ce procédé ne décrit pas une étape de diffusion moléculaire en mode statique permettant d'améliorer la biodisponibilité de la substance active.

Tom et al. (Applications of supercritical fluids in controlled release of drugs, Supercritical Fluids Engineering Science ACS Symp. Ser. 514, American Chemical Society, Washington DC, 1992) rapportent la première co-précipitation par procédé RESS de microparticules de substance active de lovastatine (anticholestérolémiant) complexée à un polymère, le DL-PLA. Les deux composés sont placés dans un autoclave, extraits par du $CO_2$ supercritique et précipités dans une seconde enceinte. L'inconvénient majeur d'un tel procédé est le ratio substance active / support obtenu dans le complexe. En effet, ce ratio ne peut être choisi précisément puisqu'il est déterminé par la solubilité de chacun des deux composés dans le $CO_2$ à l'état supercritique. Or la co-précipitation d'une substance active et d'un support n'est pas suffisante pour améliorer de façon conséquente la solubilité de la substance active en milieu aqueux. En outre ce procédé ne décrit pas une étape de diffusion moléculaire en mode statique permettant d'améliorer la biodisponibilité de la substance active, et de plus, sa solubilité dans un milieu aqueux n'est pas étudiée.

Un procédé d'imprégnation d'actifs pharmaceutiques est revendiqué dans la demande de brevet WO 99/25322 de Carli et al. Il se décompose de la manière suivante :

1. Solubilisation du principe actif par procédé RESS,
2. Mise en contact du fluide supercritique contenant le principe actif avec le polymère réticulé,
3. Imprégnation du polymère réticulé en mode statique ou dynamique,
4. Elimination du fluide supercritique.

Seules des substances actives solubles dans le fluide supercritique peuvent être préparées par ce procédé, puisque la première étape consiste en l'extraction du principe actif par le fluide supercritique. Par ailleurs, le procédé n'est pas un procédé d'inclusion mais d'imprégnation sur un support, et aucun résultat n'est donné concernant l'amélioration de la dissolution dans un milieu aqueux du principe actif ainsi préparé. Enfin, le polymère imprégné ne subit pas d'étape de lavage par fluide supercritique.

Fisher et Müller décrivent dans leur brevet US 5 043 280 un procédé de préparation de substances actives sur un support par fluide supercritique. Ce procédé consiste à mettre en contact un ou plusieurs actif(s) avec un ou plusieurs support(s) en milieu supercritique. Pour ce faire les actifs et les supports sont soit précipités, soit co-précipités par procédés SAS et/ou RESS. Les composés sont obtenus sous forme stérile. Or la précipitation ou co-précipitation d'une substance active et d'un support n'est pas suffisante pour améliorer de façon conséquente la solubilité de la substance active en milieu aqueux. En outre ce procédé ne décrit pas une étape de diffusion moléculaire en mode statique permettant d'améliorer la biodisponibilité de la substance active, et de plus, sa solubilité dans un milieu aqueux n'est pas étudiée.

Van Hees et al. (Application of supercritical carbon dioxide for the preparation of a Piroxicam-β-cyclodextrin inclusion compound, Pharmaceutical Research, Vol. 16. N°12, 1999) décrivent dans leur publication un procédé d'inclusion de Piroxicam dans les β-cyclodextrines par $CO_2$ supercritique. Le procédé consiste à placer un mélange de Piroxicam et de β-cyclodextrines (ratio molaire 1/2,5) dans un autoclave pressurisé, laissé en mode statique. Après dépressurisation le mélange obtenu est broyé et homogénéisé avant caractérisation.

Ces analyses permettent de conclure quant au taux de complexation du Piroxicam avec la β-cyclodextrine, mais ne donnent aucun résultat sur l'amélioration de la dissolution en milieu aqueux du complexe Piroxicam / β-cyclodextrine par rapport au Piroxicam seul. De plus, la substance active utilisée n'a pas été générée par fluide supercritique et aucune étape de lavage par fluide supercritique du complexe n'est réalisée.

Kamihira M. et al. (Formation of inclusion complexes between cyclodextrins and aromatic compounds under pressurized carbon dioxide, J. of Fermentation and Bioengineering, Vol. 69, N°6, 350-353, 1990) décrivent un procédé d'extraction de composés aromatiques volatiles, et de piégeage par inclusion dans les cyclodextrines. Le géraniol et l'huile de moutarde sont ainsi extraits par un procédé RESS, et vaporisés en mode dynamique dans un second autoclave contenant un mélange de cyclodextrine et d'eau. L'influence des paramètres température, pression et teneur en eau est étudiée par mesure du taux d'inclusion des substances actives dans les cyclodextrines. L'étape d'inclusion décrite dans cette publication est réalisée en mode dynamique et non statique comme revendiquée dans la présente invention. Par ailleurs ce procédé ne comprend pas d'étape de lavage par fluide supercritique. Enfin, la solubilité de la substance active dans un milieu aqueux n'est pas étudiée.

Sze Tu L. et al. (Application of dense gazes in pharmaceutical processing, Proceedings of 5th meeting on supercritical fluids, Nice, France, March 1998) décrivent dans leur publication comment pratiquer une précipitation par SAS d'une substance active (acide parahydrobenzoïque) et de polymères (PLGA - polylactictide co-glycolide - ou le PLA - poly L-lactic acid). Cette co-précipitation est réalisée suivant deux techniques; soit avec le polymère et la substance active dans deux solutions différentes; ou bien dans la même solution. Dans les deux cas, les deux solutions, ou la solution, contenant les deux composants sont traitées par SAS $CO_2$ supercritique. Or, la co-précipitation d'une substance active et d'un support poreux n'est pas suffisante pour améliorer de façon conséquente la solubilité de la substance active en milieu aqueux. En outre, cette méthode ne décrit pas une étape de diffusion moléculaire en mode statique permettant d'améliorer la biodisponibilité de la substance active, et, de plus, sa solubilité dans un milieu aqueux n'est pas étudiée.

Il en est de même pour les procédés de co-précipitation décrits par Jung et al. dans leur brevet FR 2 815 540. Il s'agit d'un procédé de fabrication de très fines particules contenant au moins un principe actif inséré dans une molécule hôte, ainsi qu'un dispositif permettant la mise en oeuvre de ce procédé. Ce procédé consiste à mettre en solution le principe actif dans un premier solvant liquide, et un produit formé des molécules hôtes, de type cyclodextrines ou éther-couronne, dans un second solvant liquide. Les solutions sont alors mises en contact avec un fluide à pression supercritique, de façon à faire précipiter les molécules, selon un procédé SAS. Les composants, comme dans le procédé décrit par Sze Tu L. dans l'article cité précédemment, peuvent être solubilisés dans le même solvant. Les résultats présentés par Jung et al. ne revendiquent pas d'amélioration de la vitesse de dissolution. Or, la co-précipitation d'une substance active et d'un support de type cyclodextrine n'est pas suffisante pour améliorer de façon conséquente la solubilité de la substance active en milieu aqueux. En outre, cette méthode ne décrit pas une étape de diffusion moléculaire en mode statique permettant d'améliorer la biodisponibilité de la substance active, et, de plus, sa solubilité dans un milieu aqueux n'est pas étudiée.

De façon surprenante les inventeurs de la présente demande ont découvert qu'un procédé comprenant les étapes de génération d'une substance active peu soluble dans un milieu aqueux par un fluide supercritique, son mélange avec un support poreux suivi d'une étape de diffusion moléculaire par le fluide supercritique en mode statique et du lavage par le fluide supercritique permettait de préparer un composé d'interaction en augmentant très fortement la solubilité dans un milieu aqueux de la substance active, et donc sa biodisponibilité.

En effet, l'étape d'inclusion en mode statique couplée à la phase de précipitation de la substance active à son support a permis de façon surprenante d'améliorer la dissolution de la substance active en milieu aqueux. De plus, la troisième phase de lavage en milieu supercritique, qui consiste à éliminer les solvants résiduels par passage d'un flux de $CO_2$ supercritique permet également, de façon surprenante, outre le lavage du composé d'interaction, d'augmenter la dissolution consécutive à cette étape.

De plus, ces étapes peuvent être réalisées en batch ou en continu, comme c'est notamment le cas pour la diffusion et le lavage. Cela permet donc d'alléger le procédé par rapport aux étapes conventionnelles qui seraient :

1. Cristallisation
2. Séparation solide / liquide
3. Séchage
4. Inclusion dans le support
5. Micronisation

Ainsi, la présente invention concerne un procédé de préparation d'un composé d'interaction d'une substance active peu soluble dans un milieu aqueux avec un support poreux, caractérisé en ce qu'il comprend les étapes suivantes :

(a) Mélanger avantageusement de façon intime la substance active générée par fluide supercritique et la quantité déterminée de support poreux,
(b) Mettre en oeuvre une étape de diffusion moléculaire par mise en contact en mode statique d'un fluide supercritique avec le mélange obtenu à l'étape (a) pendant le temps nécessaire pour améliorer la dissolution dans un milieu aqueux du mélange obtenu à l'étape (a),
(c) Laver le composé d'interaction obtenue à l'étape (b) par un flux de fluide supercritique,
(d) Récupérer les particules du composé d'interaction ainsi formé.

Par « substance active peu soluble dans un milieu aqueux », on entend au sens de la présente invention toute substance active peu ou pas soluble dans un milieu aqueux et ayant en particulier une solubilité inférieure à au moins 20 μg/ml. En particulier il peut s'agir d'un actif pharmaceutique, cosmétique ou nutraceutique. Avantageusement il s'agit d'une substance active choisie dans le groupe constitué par les dérivés d'anilide, les dérivés d'épipodophyllotoxine, le piroxicam, l'acide valérique, l'acide octanoïque, l'acide laurique et l'acide stéarique. Dans le cas des dérivés d'anilides, il s'agit de façon avantageuse d'un dérivé de formule générale I suivante :

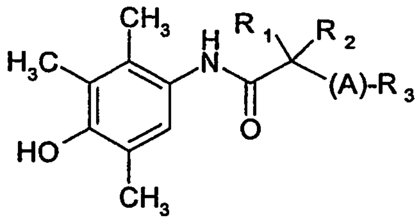

I

dans laquelle :

$R_1$ et $R_2$ identiques ou différents représentent indépendamment l'un de l'autre un atome d'hydrogène ; un radical alcoyle linéaire ou ramifié en $C_1$-$C_6$ ; un groupement aromatique tel que phényle, naphtyle ou pyridyle éventuellement substitué par un ou plusieurs groupements alcoyle en $C_1$-$C_4$, alcoxy en $C_1$-$C_4$, hydroxyle ou halogéno,
$R_3$ représente une chaîne alcoyle linéaire ou ramifiée en $C_6$-$C_{15}$ ou un groupement phényle éventuellement substitué par un ou plusieurs groupements alcoyle en $C_1$-$C_4$, alcoxy en $C_1$-$C_4$, hydroxyle ou halogéno,
A représente un atome de soufre ou d'oxygène ou le groupement sulfoxy.

De façon encore plus avantageuse, il s'agit du (S)-2',3',5'-triméthyl-4'- hydoxy-α-dodécylthiophényl acétanilide (F12511). Les composés de formule I pouvant posséder des centres d'asymétrie, la substance active selon la présente invention peut être un des différents stéréoisomères ou énantioméres ou leur mélange. Ces dérivés et leur mode de préparation sont décrits dans la demande de brevet FR2741619.

Dans le cas des dérivés d'épipodophyllotoxine, il s'agit de façon avantageuse d'un dérivé de formule générale II suivante

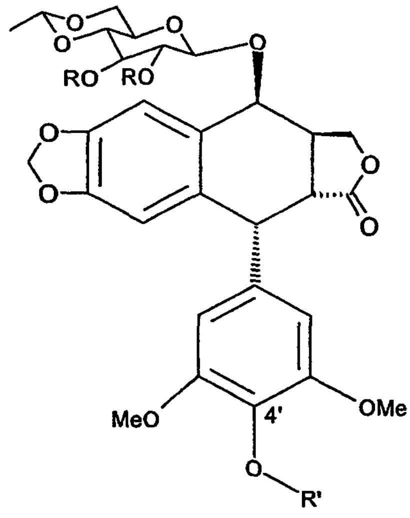

II

dans laquelle

R' représente un atome d'hydrogène ; un groupement phosphate monoester ; un groupement carbamate de type -CO-N($R_1R_2$) où N($R_1R_2$) représente des groupements aminodiacétiques et une amine polycyclique comme la 3-amino-quinuclidine ; un groupement acyle de type phosphonoacétique $H_2O_3P$-$CH_2$-CO ou un radical R,
R représente un groupe acyle de formule A-Z-$CH_2$-CO où Z représente un atome d'oxygène, de soufre, un groupement $SO_2$, un alkylène linéaire ou ramifié en $C_{1-4}$, dans ce cas A représente un noyau phényle substitué ou non, à la condition que

- dans le cas où R=R', c'est à dire les dérivés triacylés, A représente un noyau aromatique possédant une fonction salifiable,
- dans le cas où R'≠R, A représente un reste benzyle, naphtyle, hétéroaryle, phényle substitué ou non, dans ce cas le phényle pouvant être substitué une ou plusieurs fois quelle que soit sa position sur le noyau aromatique par des groupes tels que halogènes, F, Cl, Br, alcoxy linéaire ou cyclique en $C_{1-6}$, alkyle en $C_{1-6}$, méthylène dioxy, $OCF_3$, $CF_3$, $NO_2$, CN, $OCH_2$ Aryle, OH, $OPO_3H_2$, $CH_2PO_3H_2$, $PO_3H_2$, $OCH_2CO_2H$, COOH, $CH_2COOH$, $COCH_3$, CHO,

A-Z peut également représenter un groupement $OCH_2CO_2H$, $SO_2CH_2COOH$, $PO_3H_2$.

De façon encore plus avantageuse, il s'agit du 4'-déméthyl-4'-désoxy-4'-phosphate-4-0-(2,3-bis-(2,3,4,5,6-pentafluorophénoxyacétyl)-4,6-éthylidène-β-D-glucosyl)-épipodophyllotoxine (L0081).

Ces dérivés et leur mode de préparation sont décrits dans la demande de brevet FR2725990.

Par « substance active générée par fluide supercritique», on entend au sens de la présente invention, toute substance active telle que définie ci-dessus qui a subi une étape de génération par fluide supercritique, c'est à dire une étape permettant grâce à l'utilisation du fluide supercritique d'augmenter sa surface spécifique. Avantageusement une telle étape consiste en un procédé RESS ou SAS.

Par « support poreux », on entend au sens de la présente invention tout support poreux approprié soluble dans un milieu aqueux. Avantageusement le support poreux est choisi dans le groupe constitué par les cyclodextrines et leur mélange. De façon avantageuse, il s'agit de la γ-cyclodextrine.

Par « fluide supercritique », on entend au sens de la présente invention tout fluide utilisé à une température et une pression supérieure à leur valeur critique. Avantageusement il s'agit du $CO_2$.

Par « Mode statique » on entend au sens de la présente invention une réaction ou un procédé dans lequel tous les réactifs sont mis simultanément en présence et où on laisse la réaction se dérouler. Par exemple, dans l'étape (b) de la présente invention, on met dans un autoclave une poudre co-cristallisée, de l'eau et du $CO_2$ supercritique et on laisse réagir pendant 16 Heures. La masse de produit n'évolue pas durant la réaction.

A l'inverse, en mode dynamique, les réactifs sont apportés au fur et à mesure de l'évolution de la réaction ou de la production. Souvent dans le cadre d'un mode dynamique, il y a circulation d'un fluide ou agitation. La masse de

produit évolue durant la production. Dans le procédé de la présente invention, l'étape (a) est typiquement une phase dynamique.

Par « mélange intime », on entend au sens de la présente invention un mélange de A et B dans lequel A et B se retrouvent uniformément réparties au sein du mélange obtenu.

Dans un mode de réalisation particulier, le procédé selon la présente invention est tel que le support poreux est généré par fluide supercritique et que l'étape (a) comprend les étapes suivantes :

(a1) Mettre en solution la substance active et le support poreux dans un solvant organique, ledit solvant organique étant soluble dans le fluide supercritique,
(a2) Mettre en contact de façon continue la solution obtenue à l'étape (a1) avec ledit fluide supercritique, afin de désolvater de façon contrôlée la substance active et le support, et assurer leur coacervation,
(a3) Laver le complexe ainsi formé par extraction du solvant résiduel par le fluide supercritique, puis procéder à la séparation du solvant à l'état liquide et du fluide supercritique à l'état gazeux.

Avantageusement l'étape (a) consiste en une coprécipitation de la substance active et du support poreux par le procédé SAS.

Dans un autre mode de réalisation, le procédé selon la présente invention est tel que la substance active, avant son utilisation dans l'étape (a), est générée par le procédé comprenant les étapes suivantes :

(i) Mettre en solution la substance active dans un solvant organique, ledit solvant organique étant soluble dans le fluide supercritique,
(ii) Mettre en contact de façon continue la solution obtenue à l'étape (i) avec ledit fluide supercritique, afin de désolvater la substance active, et assurer sa coacervation,
(iii) Laver les particules de substance active ainsi formées par extraction du solvant résiduel par ledit fluide supercritique, puis procéder à la séparation du solvant à l'état liquide et du fluide supercritique à l'état gazeux,

et que le support poreux utilisé à l'étape (a) est sous forme solide.

Avantageusement la substance active, avant son utilisation dans l'étape (a), est générée par précipitation selon le procédé SAS.

Dans un troisième mode de réalisation, le procédé selon la présente invention est tel que la substance active, avant son utilisation dans l'étape (a) est générée par le procédé comprenant les étapes suivantes :

(i) Extraire la substance active par le fluide supercritique, éventuellement additionné d'un co-solvant,
(ii) Vaporiser le mélange supercritique afin de désolvater la substance active, et assurer sa coacervation,
(iii) Laver les particules de substance active ainsi formées par le fluide supercritique, puis éventuellement procéder à la séparation du co-solvant à l'état liquide et du fluide supercritique à l'état gazeux,

et que le support poreux utilisé à l'étape (a) est sous forme solide.

Avantageusement, la substance active, avant son utilisation dans l'étape (a), est générée par précipitation selon le procédé RESS.

Dans un quatrième mode de réalisation, le procédé selon la présente invention est tel que l'étape (a) comprend les étapes suivantes :

(a1) Mettre en solution la substance active dans un solvant organique, ledit solvant organique étant soluble dans le fluide supercritique,
(a2) Mettre en contact de façon continue la solution ainsi obtenue avec le fluide supercritique, afin de désolvater la substance active, et assurer sa coacervation sur le support poreux préalablement placé dans le réacteur,
(a3) Laver le complexe ainsi formé par extraction du solvant résiduel par le fluide supercritique, puis procéder à la séparation du solvant à l'état liquide et du fluide supercritique à l'état gazeux.

Avantageusement, l'étape (a) consiste en la précipitation par le procédé SAS de la substance active sur le support poreux.

Dans un cinquième mode de réalisation, le procédé selon la présente invention est tel que l'étape (a) comprend les étapes suivantes :

(a1) Extraire la substance active par un fluide supercritique, éventuellement additionné d'un co-solvant,
(a2) Vaporiser le mélange supercritique afin de désolvater la substance active, et assurer sa coacervation sur le support poreux préalablement placé dans le réacteur,
(a3) Laver le complexe ainsi formé par le fluide supercritique, puis éventuellement procéder à la séparation du co-solvant à l'état liquide et du fluide supercritique à l'état gazeux.

Avantageusement, l'étape (a) consiste en la précipitation par le procédé RESS de la substance active sur le support poreux.

De façon avantageuse, le solvant organique ou le co-solvant est choisit dans le groupe constitué par les alcools, en particulier le méthanol ou le butanol, les cétones, en particulier l'acétone, la méthyléthylcétone, la cyclohéxanone ou la N-méthylpyrrolidone, l'acide acétique, l'acétate d'éthyle, le dichlorométhane, l'acétonitrile, le diméthylformamide, le diméthylsulfoxyde (DMSO) et leur mélange. Avantageusement il s'agit de l'éthanol ou du diméthylsulfoxyde.

De façon avantageuse, l'étape (b) de diffusion moléculaire du procédé selon la présente invention est réalisée sous agitation.

De façon encore plus avantageuse, l'étape (b) de diffusion moléculaire du procédé selon la présente invention est réalisée en présence d'un agent de diffusion.

Par « agent de diffusion », on entend au sens de la présente invention n'importe quel solvant favorisant une interaction de la substance active avec le support.

Avantageusement, cet agent de diffusion est choisi dans le groupe constitué par l'alcool, l'eau avec ou sans agent surfactant et leurs mélanges. De façon encore plus avantageuse, il s'agit de l'eau.

Cet agent de diffusion peut être ajouté en continu ou en discontinu.

Le temps nécessaire à la diffusion moléculaire de l'étape (b) est déterminé par toute méthode appropriée. Cette étape (b) peut être réitérée autant de fois que souhaitée pour obtenir une vitesse de dissolution satisfaisante. Avantageusement, l'étape (b) dure environ 16 heures.

Les conditions de pression et de température de l'étape (b) sont choisies de façon à favoriser la diffusion moléculaire. Avantageusement la pression du fluide supercritique est comprise entre 10 MPa et 40 MPa et la température entre 0 et

120°C.

De façon encore plus avantageuse, le fluide supercritique est utilisé à une pression comprise entre 10 MPa et 40 MPa et à une température comprise entre 0 et 120°C dans toutes les étapes du procédé selon la présente invention.

Avantageusement chacune des étapes du procédé selon la présente invention est mise en oeuvre dans un réacteur fermé, en particulier un autoclave.

De façon avantageuse le procédé selon la présente invention est réalisé en continu.

La présente invention concerne également un composé d'interaction d'une substance active peu soluble dans un milieu aqueux avec un support poreux caractérisé en ce qu'il est susceptible d'être obtenu par le procédé selon la présente invention.

De façon avantageuse, le composé d'interaction selon la présente invention est tel que la substance active ainsi complexée présente une solubilité dans une solution aqueuse de Laurylsulfate de sodium à 5 % supérieure à environ 600 μg/ml.

<u>Caractéristiques physiques des poudres aux différentes étapes</u> :

<u>Poudre de principe actif obtenue par RESS</u> :

- poudre extrêmement légère et pulvérulente,
- taille et type de cristaux monodispersés :bâtonnets de longueur de 1-3 μm et de diamètre 100 à 200 nm,
- densité apparente de 12 kg/m$^3$.

<u>Poudre de principe actif obtenue par SAS</u> :

- Poudre très légère et pulvérulente,
- taille et type de cristaux monodispersés : bâtonnets de longueur de 10-20μm et de diamètre 100 nm,
- densité apparente 97 kg/m$^3$.

<u>Poudre co-cristallisée (principe actif/cyclodextrine)</u>

- poudre fine légère et pulvérulente,
- densité apparente 176 kg/$m^3$

Poudre co-cristallisée, maturée (principe actif/cyclodextrine)

- poudre dense et non pulvérulente,
- densité apparente 639 kg/$m^3$.

D'autres objets et avantages de l'invention deviendront apparents pour l'homme du métier à partir de la description détaillée ci-dessous et par le biais de références aux dessins illustratifs suivants.

La figure 1 représente une photo MEB avec un agrandissement de 1000x du produit F12511 obtenu après cristallisation et séchage par voie classique.
La figure 2 représente une photo MEB avec un agrandissement de 2000x du produit F12511 obtenu après cristallisation et séchage par voie classique.
La figure 3 représente une photo MEB avec un agrandissement de 1000x du complexe obtenu après co-précipitation par le procédé SAS et lavage par $CO_2$ supercritique d'une solution du produit F12511 et de γ-cyclodextrine dans le DMSO.
La figure 4 représente une photo MEB avec un agrandissement de 2000x du complexe obtenu après co-précipitation par le procédé SAS et lavage par $CO_2$ supercritique d'une solution du produit F12511 et de γ-cyclodextrine dans le DMSO.
La figure 5 représente une photo MEB avec un agrandissement de 1000x du même complexe que les figures 3 et 4 après 16 heures de diffusion moléculaire en milieu supercritique, en présence d'eau.
La figure 6 représente une photo MEB avec un agrandissement de 2000x du même complexe que les figures 3 et 4 après 16 heures de diffusion moléculaire en milieu supercritique, en présence d'eau.
La figure 7 représente un histogramme de la biodisponibilité du produit F12511 selon la formulation utilisée (composé d'interaction avec le γ-cyclodextrine selon le procédé de la présente invention ou produit F12511 cristallisé) chez le chien.

Le procédé selon l'invention comprend notamment une étape de diffusion moléculaire en milieu supercritique permettant une forte interaction des particules de substance active dans le support envisagé, comme le montrent les photos réalisées au microscope à balayage électronique (figures 1 à 6). On peut voir sur ces photos que la structure du composé est totalement modifiée au cours de la diffusion. De plus, la dissolution en milieu aqueux est également modifiée.
Ainsi, le composé selon les figures 1 et 2 a une solubilité au bout de 2 heures de 6 μg/ml dans une solution aqueuse à 5% de laurylsulfate de sodium.
Le complexe selon les figures 3 et 4 a une solubilité au bout de 2 heures de 86 μg/ml dans une solution aqueuse à 5% de laurylsulfate de sodium.
Le complexe selon les figures 5 et 6 a une solubilité au bout de 2 heures de 516 μg/ml dans une solution aqueuse à 5% de laurylsulfate de sodium.
L'objectif recherché au cours de cette étape de diffusion est d'améliorer la dissolution des micro-particules de substance active.
L'étape suivante qui est une étape de lavage par fluide supercritique, permet encore d'augmenter la vitesse de dissolution du composé d'interaction de la substance active dans le support poreux.
La dissolution au bout de deux heures dans un milieu aqueux est multipliée par environ 100 par le procédé selon la présente invention.
Les exemples suivant de mise en oeuvre du procédé sont donnés à titre indicatif non limitatif.

**Protocoles d'analyse des poudres**

*Tests de dissolution du produit F12511*

Conditions opératoires :

Détecteur spectrophotométrique réglé à 220 nm.
Colonne greffage C8 (Lichrospher 60RP-Select B), dimensions 25 x 0,4 cm, granulométrie : 5 μm.

Phase mobile :

| | |
|---|---|
| * Acétonitrile | 820 ml |
| * Eau purifiée | 180 ml |
| * Acide acétique glacial | 1 ml |

Débit : 1 ml/min

Préparation des solutions :

***Solution à examiner***

Introduire une quantité de complexe correspondant à environ 100 mg du produit F12511 dans 100 ml de lauryl sulfate de sodium à 5% (m/V) dans $H_2O$. Placer sous agitation magnétique dans un bain-marie à 37°C ± 0,5°C. Prélever 2 ml de cette suspension après 2 heures d'agitation et filtrer sur filtre GELMAN GHP ACRODISC GF (R).
Diluer les prélèvements au 1/5 dans la phase mobile.
Effectuer 2 essais.

***Solution témoin***

Introduire 8 mg du produit F12511 de référence (matière première ayant servi à la fabrication du complexe) dans une fiole de 100 ml, dissoudre dans 1 ml de tétrahydrofurane (THF).
Compléter au volume avec la phase mobile.

***Gamme***

| | T1 | T2 | T3 | T4 | T5 |
|---|---|---|---|---|---|
| Solution témoin (ml) | 0,5 | 1,5 | 2,0 | 3,0 | 4,0 |
| Phase mobile | qsp 20 ml | | | | |
| Concentration (μg/ml) | 2,0 | 6,0 | 8,0 | 12,0 | 16,0 |

Réalisation de l'essai :

Injecter 20 μl de chaque solution témoin. Mesurer l'aire du pic du produit F12511 et représenter graphiquement sa variation en fonction de la concentration. Le coefficient de corrélation est >0,995. Injecter 20 μl de la solution essai. Mesurer l'aire du pic du produit F12511 présent dans la solution essai, et s'assurer qu'elle est comprise entre celle du T1 et du T5 de la gamme.

Dans le cas contraire, effectuer une dilution dans le solvant de solubilisation et/ou ajuster le volume d'injection de la solution essai.

En déduire la concentration X (μg/ml) de la solution essai.

Calculer la quantité du produit F12511 solubilisé en mg/ml par la formule :

$$\frac{X \times 20 \times F \times 5}{1000 \times Y}$$

Y : volume d'injection de la solution essai
F : facteur de dilution

Mesures de surfaces spécifiques

Les mesures de surface spécifique ont été effectuées sur un appareil à adsorption BET ASAP 2010, Micrometrics.

Préparation de l'échantillon

Avant la phase de mesure, l'échantillon nécessite une étape de dégazage. Cette étape consiste à faire le vide dans la cellule contenant l'échantillon jusqu'à ce que l'on ait atteint au moins un vide de 0,003 mm Hg, soit 0,004 mbar environ, et ceci de manière stable. Ce dégazage est réalisé à une température de 50°C (durée : environ 16 heures).
En fin de dégazage, la cellule contenant l'échantillon est remplie d'hélium, et transférée au poste de mesure où l'on refait le vide avant analyse.

Exploitation des isothermes d'adsorption

La détermination de la surface spécifique a été faite selon la théorie BET, soit selon la relation :

$$\frac{1}{W.[(P_0/P)-1]} = \frac{1}{CWm} + \frac{C-1}{Wm.C}.(P/P_0)$$

W : volume de gaz adsorbé (dans les conditions standard de température et de pression (STP)) par unité de masse d'échantillon.
Wm : volume de gaz adsorbé (dans les conditions STP) dans une monocouche par unité de masse d'échantillon.
$P_0$ : pression de saturation.
C : constante.

On retrace alors l'isotherme selon :

$$\frac{1}{W.[(P_0/P)-1]}$$

En fonction de $P/P_0$ : nous avons alors une droite dont la pente et l'ordonnée à l'origine nous donnent C et Wm.
La surface spécifique est alors donnée par la formule :

$$a\ (m^2.g^{-1}) = N_m N_A E$$

E : encombrement de la molécule d'azote. On prend généralement pour l'azote à 77
K, température opératoire, E = 0,162 $nm^2$.
$N_A$ : nombre d'Avogadro.
$N_m$ : nombre de moles d'azote adsorbées sur une monocouche par unité de masse d'échantillon, calculé à partir de Wm.

Les mesures sont réalisées dans un domaine classique de pression relative où la théorie BET est valable, soit $0,05 < P/P_0 < 0,2$. Pour vérifier la validité de cette théorie, un moyen pratique est de regarder dans quel sens évolue la quantité $N_{adsorbé}$ . $(1-P/P_0)$ en fonction de $P/P_0$: elle doit continuellement augmenter avec $P/P_0$. Vérifier de cette manière le domaine d'applicabilité de la théorie BET, et réajuster si besoin est, le domaine des pressions relatives.

**Exemple comparatif 1 : précipitation par SAS / DMSO du produit F12511**

Une solution de 150 ml de concentration : 115 g/l du produit F12511 dans le DMSO, est précipitée en continu par le procédé Solvant-Anti-Solvant (SAS) en présence de $CO_2$, dans un autoclave de 21 muni d'un panier de 1,371. Le débit de la pompe solvant est de 0,6 ml/min. La température et la pression au sein de l'autoclave sont choisies pour obtenir une densité de $CO_2$ égale à 0, 8. Après avoir précipité environ 130 ml de solution, les injections du soluté puis de $CO_2$ sont arrêtées, et l'on procède au lavage par passage d'un flux de $CO_2$ (300 bars, 50°c) pendant 3 heures. L'autoclave est ensuite dépressurisé. Le rendement de cette étape est de 87 %.

| Nature de la poudre | Dissolution (μg/ml) | BET ($m^2/g$) |
|---|---|---|
| F12511 | 6-12 | 14 |
| F 12511 précipité par SAS | 62 | 54 |

**Exemple comparatif 2 : précipitation par RESS du produit F12511**

On place 10 g de produit F12511 dans un autoclave, que l'on extrait par du $CO_2$ supercritique à 100°C, 265 bars. Le fluide est alors précipité dans une deuxième enceinte, et l'on récupère 0,6 g de produit F12511. On mesure la dissolution au bout de deux heures ainsi que la surface spécifique :

| Nature de la poudre | Dissolution (μg/ml) | BET ($m^2/g$) |
|---|---|---|
| F12511 | 12 | 14 |
| F 12511 précipité par RESS | 76 | 67 |

**Exemple comparatif 3: co-précipitation du produit F12511 et de la γ-cyclodextrine par SAS / DMSO**

Une solution de 150 ml de produit F12511 (concentration : 57,5 g/l) et de γ-cyclodextrine (concentration de 172,5 g/l) dans le DMSO, est précipitée en continu par le procédé Solvant-Anti-Solvant (SAS) en présence de $CO_2$, dans un autoclave de 21 muni d'un panier de 1,371. Le débit de la pompe solvant est de 0,4 ml/min. La température et la pression au sein de l'autoclave sont choisies pour obtenir une densité de $CO_2$ égale à 0,9. Après avoir précipité environ 100 ml de solution, les injections du soluté puis de $CO_2$ sont arrêtées, et l'on procède au lavage de la poudre obtenue par passage d'un flux de $CO_2$ (300 bars, 50°C) pendant 2 heures. L'autoclave est ensuite dépressurisé.

Le rendement de cette étape est de 81 %.

Les résultats des mesures de dissolution sont rassemblés dans le tableau ci-dessous :

| Nature de la poudre | Dissolution (μg/ml) |
|---|---|
| F 12511 | 12 |
| F 12511 co-précipité par SAS/DMSO | 100 |

**Exemple 4 : co-précipitation, inclusion et lavage à partir d'une solution du produit F12511 et de γ-cyclodextrine dans le DMSO**

Une solution de 450 ml du produit F12511 (concentration : 40 g/l) et de γ-cyclodextrine (concentration de 240 g/l) dans le DMSO, est précipitée en continu par le procédé Solvant-Anti-Solvant (SAS) en présence de $CO_2$, dans un autoclave de 6l muni d'un panier de 4l. Le débit de la pompe solvant est de 1,1 ml/min. La température et la pression au sein de l'autoclave sont choisies pour obtenir une densité de $CO_2$ égale à 0,9 ± 0,05. Après avoir précipité environ 450 ml de solution,

les injections du soluté puis de $CO_2$ sont arrêtées, et l'on procède à la détente douce de l'installation, de façon à ne pas liquéfier le fluide supercritique.

Le rendement moyen de cette étape est de 94%.

On mélange la poudre co-précipitée à l'étape précédente avec de l'eau osmosée (ratio massique de 25% d'eau), et le mélange est placé dans le panier poral de 4L, lui-même déposé dans l'autoclave de précipitation de 6 1.

L'autoclave est fermé, et l'on gonfle l'installation en $CO_2$ supercritique de façon à avoir une pression statique de 300 bars, et une température de 65°C au sein de l'autoclave.

On procède à la détente douce après une nuit de diffusion moléculaire, et l'on réitère cette étape, sans ajout d'agent de diffusion (eau) pendant une nuit.

Puis l'on procède au lavage du complexe ainsi obtenu par flux de $CO_2$ supercritique (270 bars, 40°C) pendant 8 heures. On effectue après détente une mesure de dissolution sur la poudre obtenue.

| Nature de la poudre | Dissolution (μg/ml) |
|---|---|
| F12511 avant co-précipitation | ~15 |

(suite)

| Nature de la poudre | Dissolution ($\mu$g/ml) |
|---|---|
| Composé F12511 / $\gamma$-cyclodextrine après diffusion moléculaire | 440 |
| Composé F12511 / $\gamma$-cyclodextrine après diffusion moléculaire et lavé | 662 |

Ces résultats démontrent l'intérêt d'un procédé associant la co-précipitation, l'inclusion et le lavage en milieu supercritique pour améliorer la dissolution en milieu aqueux du principe actif.

Des essais pharmacocinétiques sur le chien ont été réalisés avec un composé d'interaction F12511/$\gamma$-cyclodextrine obtenu par ce procédé. Des doses normalisées de 3 mg/kg ont été administrées à 5 chiens, et la concentration plasmatique (exprimée en ng/ml.h) en F12511 a été mesurée. Les résultats concernant le F12511 obtenu après cristallisation et séchage par voie classique et ceux concernant le composé d'interaction F12511/$\gamma$-cyclodextrine obtenu par le procédé décrit ci-dessus de la présente invention sont représentés dans l'histogramme de la figure 7.

On constate que l'administration de doses préparées à partir du composé d'interaction F12511/$\gamma$-cyclodextrine obtenu par le procédé selon la présente invention permet d'améliorer la biodisponibilité chez le chien d'un facteur 10.

**Exemple comparatif 5 : précipitation et inclusion dans la $\gamma$-cyclodextrine du produit F12511 générée par procédé SAS / Ethanol**

Une solution de 8 l du produit F12511 (concentration : 5 g/l) dans l'éthanol, est précipitée en continu par le procédé Solvant-Anti-Solvant (SAS) en présence de $CO_2$, dans un autoclave de 6l muni d'un panier de 4l. Le débit de la pompe solvant est de 41,7 ml/min. La température et la pression au sein de l'autoclave sont choisies pour obtenir une densité de $CO_2$ égale à 0,8. Après avoir précipité environ 8l de solution, les injections du soluté puis de $CO_2$ sont arrêtées, et l'on procède à la détente douce de l'installation, de façon à ne pas liquéfier le fluide supercritique.

On mélange 4,3 g de la substance active précipitée à l'étape précédente avec 25,8 g de $\gamma$-cyclodextrine et 10 g d'eau osmosée, et le mélange est placé dans le panier poral de 4l, lui-même déposé dans l'autoclave de précipitation de 6l.

L'autoclave est fermé, et l'on gonfle l'installation en $CO_2$ supercritique de façon à avoir une pression statique de 300 bars, et une température de 65°C au sein de l'autoclave.

On procède à la détente douce après 16 heures de diffusion moléculaire.

| Nature de la poudre | Dissolution (en $\mu$g/ml) |
|---|---|
| F12511 avant précipitation | ~15 |
| F12511 précipité par $CO_2$ supercritique | 80 |
| Composé F12511 / $\gamma$-cyclodextrine après diffusion moléculaire | 155 |

**Exemple comparatif 6 : précipitation et inclusion dans la $\gamma$-cyclodextrine du produit F12511 générée par procédé SAS / DMSO**

Une solution de 150 ml du produit F12511 (concentration : 200 g/l) dans le DMSO, est précipitée en continu par le procédé Solvant-Anti-Solvant (SAS) en présence de $CO_2$, dans un autoclave de 2l muni d'un panier de 1,37l. Le débit de la pompe solvant est de 0,5 ml/min. La température et la pression au sein de l'autoclave sont choisies pour obtenir une densité de $CO_2$ égale à 0,9. Après avoir précipité environ 135 ml de solution, les injections du soluté puis de $CO_2$ sont arrêtées, et l'on procède à la détente douce de l'installation, de façon à ne pas liquéfier le fluide supercritique.

On mélange 1 g de la substance active précipitée à l'étape précédente avec 6 g de $\gamma$-cyclodextrine et 2,33 g d'eau osmosée, et le mélange est placé dans le panier poral de 1,371, lui-même déposé dans l'autoclave de précipitation de 2l.

L'autoclave est fermé, et l'on gonfle l'installation en $CO_2$ supercritique de façon à avoir une pression statique de 300 bars, et une température de 100°C au sein de l'autoclave.

On procède à la détente douce après 16 heures de diffusion moléculaire.

| Nature de la poudre | Dissolution (en $\mu$g/ml) |
|---|---|
| F12511 avant -précipitation | 5 |

(suite)

| Nature de la poudre | Dissolution (en $\mu$g/ml) |
|---|---|
| F12511 précipité par $CO_2$ supercritique | 57 |
| Composé F12511 / $\gamma$-cyclodextrine après diffusion moléculaire | 165 |

**Exemple comparatif 7 : Inclusion dans la $\gamma$-cyclodextrine du produit F12511 générée par procédé RESS**

On place 40 g du produit F12511 dans un panier de 4l, lui-même déposé dans un autoclave de 6l. La substance active est extraite par un mélange supercritique de $CO_2$ et d'éthanol (5% massique) et la substance est précipitée à 120 bar et 55°C. Après 3 heures, les injections de $CO_2$ et d'éthanol sont arrêtées.

On mélange 8,96 g de la substance active précipitée à l'étape précédente avec 53,76 g de $\gamma$-cyclodextrine et 20,87 g d'eau osmosée, et le mélange est placé dans le panier poral de 4l, lui-même déposé dans l'autoclave de précipitation de 6l.

L'autoclave est fermé, et l'on gonfle l'installation en $CO_2$ supercritique de façon à avoir une pression statique de 300 bars, et une température de 65°C au sein de l'autoclave.

On procède à la détente douce après 16 heures de diffusion moléculaire.

| Nature de la poudre | Dissolution (en $\mu$g/ml) |
|---|---|
| F12511 avant précipitation | ~10 |
| F12511 précipité par $CO_2$ supercritique | 8 |
| Composé F12511 / $\gamma$-cyclodextrine après diffusion moléculaire | 292 |

**Exemple comparatif 8 : Inclusion dans la $\gamma$-cyclodextrine du produit L0081 par diffusion moléculaire agitée**

On mélange 4,0 g du produit L0081, 24,0 g de $\gamma$-cyclodextrine, et 9,3 g d'eau.

Le mélange ainsi obtenu est placé au fond d'un autoclave agité d'1litre. L'autoclave est fermé hermétiquement, puis gonflé à 300 bars avec du $CO_2$ à l'état supercritique. La température a été fixée à 50°C $\pm$10°C. L'agitation est mise en route (400 tr/min), la pression et la température sont maintenues pendant une nuit. Après une nuit, on coupe le chauffage et l'agitation, et on dépressurise lentement l'autoclave La totalité de la poudre est récupérée, et l'on procède à des tests de dissolution que l'on compare avec ceux de la poudre obtenue dans les mêmes conditions mais sans agitation :

| Nature de la poudre | Dissolution (en $\mu$g/ml) |
|---|---|
| Composé L0081/ $\gamma$-cyclodextrine obtenue par diffusion moléculaire sans agitation | 124 |
| Composé L0081/ $\gamma$-cyclodextrine obtenue par diffusion moléculaire avec agitation | 334 |

**Résumé des résultats**

Le tableau ci-dessous résume les différents procédés mis en oeuvre, ainsi que les résultats de dissolution correspondants, et permet d'en déduire le procédé le plus adapté à la fabrication du produit F12511 de dissolution élevée en milieu aqueux :

| Procédé | Comp. Ex.1 | Comp. Ex.2 | Comp. Ex.3 | Ex.4 | Ex.4 | Comp. Ex.5 | Comp. Ex.5 |
|---|---|---|---|---|---|---|---|
| Précipitation* par RESS | | X | | | | | |
| Précipitation* par SAS / DMSO | X | | | | | | |
| Co-précipitation** par SAS/DMSO | | | X | X | X | | |
| Précipitation* par SAS / EtOH | | | | | | X | X |
| Cristallisation classique | | | | | | | |
| Diffusion moléculaire agitée | | | | | | | |
| Diffusion moléculaire non-agitée | | | | X | X | | X |
| Lavage | X | | X | | X | | |
| Dissolution (µg/ml) | 62 | 76 | 100 | 440 | 662 | 80 | 155 |

| Procédé | Comp. Ex.6 | Comp. Ex.6 | Comp. Ex.7 | Comp. Ex.7 | Comp. Ex.8 | Comp. Ex.8 |
|---|---|---|---|---|---|---|
| Précipitation* par RESS | | | X | X | | |
| Précipitation* par SAS/DMSO | X | X | | | | |
| Co-précipitation** par SAS/DMSO | | | | | | |
| Précipitation* par SAS / EtOH | | | | | | |
| Cristallisation classique | | | | | X | X |
| Diffusion moléculaire agitée | | | | | | X |
| Diffusion moléculaire non-agitée | | X | | X | X | |
| Lavage | X | | | | | |
| Dissolution (µg/ml) | 57 | 165 | 8 | 292 | 124 | 334 |

*** Précipitation du produit F12511 seul**

**** Co-précipitation d'une solution du produit F12511 et de γ-cyclodextrine**

Au vu de ces résultats, il est clair que le procédé qui permet d'obtenir la plus importante dissolution du produit F12511 dans un milieu aqueux est le procédé combinant les étapes de génération du produit F12511 par fluide supercritique, avantageusement par co-précipitation du produit F12511 et de la γ-cyclodextrine, diffusion moléculaire en mode statique, avantageusement sous agitation, lavage.

**Essais comparatifs 9:**

Afin de valider le fait que c'est bien le procédé au complet qui nous permet d'obtenir les résultats finaux et non une des étapes intermédiaires, nous avons réalisé des tests de dissolution comme décrit précédemment sur différents mélanges et nous avons obtenu les résultats suivants:

| | Avant diffusion | Après diffusion |
|---|---|---|
| **F12511/γ-cyclodextrine Poudres brutes Mélange physique** | 19 µg/ml | 142 µg/ml |

(suite)

| | Avant diffusion | Après diffusion |
|---|---|---|
| **F12511/γ-cyclodextrine Poudres cristallisées par SAS Séparément Mélange physique** | 69 μg/ml | 150 μg/ml |
| **F12511/γ-cyclodextrine Poudres co-cristallisées** | 100 μg/ml | 671 μg/ml |

**Essai avec d'autres substances actives, avec d'autres supports et avec d'autres solvants :**

Afin de valider les résultats obtenus avec le F12511, différentes molécules appartenant à différentes classes thérapeutiques ont été testées :

| Principe Actif | Classe thérapeutique |
|---|---|
| Kétoprofène | Anti-inflammatoire |
| Oméprazole | Anti-ulcère |
| Simvastatine | Hypo-Cholestérolémiant |
| Terfénadine | Anti-histaminique |

**Conditions opératoires de fabrication des poudres étudiées :**

On applique le procédé suivant pour chacune des poudres étudiées :

- Mise en solution du principe actif et de la cyclodextrine étudiés dans le solvant.
- Mélange intime du principe actif et de la cyclodextrine étudiés par SAS en présence de $CO_2$ supercritique.
- Séchage de la poudre obtenue
- Prélèvement d'un échantillon (dans certains cas),
- Mélange de la poudre avec de l'eau osmosée, puis inclusion sous $CO_2$ à pression supercritique.
- Séchage de la poudre obtenue,
- Prélèvement d'un échantillon.

Les tests sur ces nouvelles molécules ont été réalisés avec différents solvants et différents types de support. Les différents essais réalisés sont repris dans le tableau suivant :

| Principe Actif | Cyclodextrine | Solvant | Concentration en principe actif (g/l) |
|---|---|---|---|
| Kétoprofène | β | DMSO | 25 |
| Oméprazole | γ | DMSO | 33 |
| | γ | DMF | 30 |
| Simvastatine | γ | DMSO | 25 |
| | γ | DMF | 15 |
| Terfénadine | β | DMSO | 16 |
| | β | DMF | 30 |
| | Méthyl-β | Ethanol | 8 |

**Exemple 10 : kétoprofène/ β-cylcodextrine/DMSO**

Paramètres opératoires :

| Mélange | |
|---|---|
| Durée (h) | 2 |
| Pression (MPa) | 15 |
| Température (K) | 313 |
| Ratio Molaire $CO_2$/solvant | 400 |
| Débit de la solution (ml/min) | 1 |
| **Séchage** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

Protocole d'analyse des poudres : Test de dissolution

*Conditions opératoires :*

Analyse UV à la longueur d'onde de 260 nm.

*Solution témoin :*

Préparer une solution étalon dans $H_2O$. S'assurer de maintenir une absorbance <2.

*Réalisation de l'analyse:*

Préparer 50 ml d'une solution de Kétoprofène dans de l'eau en introduisant une quantité de poudre équivalent à 50 mg de principe actif.
Laisser dissoudre sous agitation magnétique avec barreau aimanté, dans un bain marie à 37$\pm$0,5°C.
Prélever 2ml de cette suspension après 2 heures d'agitation et filtrer sur filtre GELMAN GHP 0,45 $\mu$m.
Effectuer l'analyse UV en s'assurant que l'absorbance est inférieure à 2. Dans le cas contraire, effectuer une dilution.

*Résultat obtenu :*

Après 2 heures de dissolution, les concentrations ($\mu$g/ml) mesurées sont :

| Principe actif seul | Poudre après procédé complet |
|---|---|
| 333 | 923 |

**Exemple 11 : Oméprazole / $\gamma$-cyclodextrine / DMSO :**

Paramètres opératoires :

| Mélange | |
|---|---|
| Durée (h) | 2 |
| Pression (MPa) | 15 |
| Température (K) | 313 |
| Ratio molaire $CO_2$/solvant | 400 |
| Débit de solution (ml/min) | 1 |
| **Séchages** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

Protocole d'analyses des poudres : Test de dissolution

*Conditions opératoires:*

Analyse UV à la longueur d'onde de 296 nm.

*Solution témoin :*

Préparer une solution étalon dans le lauryl sulfate de sodium à 1 % (m/v) dans $H_2O$.

S'assurer de maintenir une absorbance < 2.

*Réalisation de l'analyse :*

Préparer 50 ml d'une solution d'Oméprazole dans de l'eau en introduisant une quantité de poudre équivalent à 50 mg de principe actif.

Laisser dissoudre sous agitation magnétique avec barreau aimanté, dans un bain marie à 37$\pm$ 0,5 °C.

Prélever 2ml de cette suspension après 2 heures d'agitation et filtrer sur filtre GELMAN GHP 0,45 $\mu$m.

Effectuer l'analyse UV en s'assurant que l'absorbance est inférieure à 2. Dans le cas contraire, effectuer une dilution.

*Résultat obtenu:*

Après 2 heures de dissolution, les concentrations ($\mu$g/ml) mesurées sont :

| Principe actif seul | Poudre après procédé complet |
|---|---|
| 91 | 129 |

**Exemple 12 : Oméprazole / γ-cyclodextrine / DMF :**

Paramètres opératoires :

| **Mélange** | |
|---|---|
| Durée (h) | 2 |
| Pression (MPa) | 15 |
| Température (K) | 313 |
| Ratio molaire $CO_2$/solvant | 400 |
| Débit de solution (ml/min) | 1 |
| **Séchages** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

Protocole d'analyses des poudres : Test de dissolution

*Conditions opératoires:*

Analyse UV à la longueur d'onde de 296 nm.

*Solution témoin:*

Préparer une solution étalon dans le lauryl sulfate de sodium à 1 % (m/v) dans $H_2O$.
S'assurer de maintenir une absorbance < 2.

*Réalisation de l'analyse :*

Préparer 50 ml d'une solution d'Oméprazole dans de l'eau en introduisant une quantité de poudre équivalent à 50 mg de principe actif.
Laisser dissoudre sous agitation magnétique avec barreau aimanté, dans un bain marie à 37± 0,5 °C.
Prélever 2ml de cette suspension après 2 heures d'agitation et filtrer sur filtre GELMAN GHP 0,45 μm.
Effectuer l'analyse UV en s'assurant que l'absorbance est inférieure à 2. Dans le cas contraire, effectuer une dilution.

*Résultat obtenu:*

Après 2 heures de dissolution, les concentrations (μg/ml) mesurées sont :

| **Principe actif seul** | **Poudre après procédé complet** |
|---|---|
| 91 | 216 |

**Exemple 13: Simvastatine / γ-cyclodextrine / DMSO :**

Paramètres opératoires :

| Mélange | |
|---|---|
| Durée (h) | 2 |
| Pression (MPa) | 15 |
| Température (K) | 313 |
| Ratio molaire $CO_2$/solvant | 400 |
| Débit de solution (ml/min) | 1 |
| **Séchages** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

*Protocole d'analyse des poudres : Test de dissolution :*

*Conditions opératoires :*

Analyse UV à la longueur d'onde de 248 nm.

*Solution témoin :*

Préparer une solution étalon dans le lauryl sulfate de sodium à 1 % (m/v) dans $H_2O$.
S'assurer de maintenir une absorbance <2.

*Réalisation de l'analyse :*

Préparer 50 ml d'une solution de Simvastatine dans de l'eau en introduisant une quantité de poudre équivalente à 50 mg de principe actif.
Laisser dissoudre sous agitation magnétique avec barreau aimanté, dans un bain marie à 37± 0,5°C.
Prélever 2ml de cette suspension après 2 heures d'agitation et filtrer sur filtre GELMAN GHP 0,45 μm.
Effectuer l'analyse UV en s'assurant que l'absorbance est inférieure à 2. Dans le cas contraire, effectuer une dilution.

*Résultat obtenu :*

Après 2 heures de dissolution, les concentrations (μg/ml) mesurées sont :

| Principe actif seul | Poudre après mélange | Poudre après procédé complet |
|---|---|---|
| < 1 | 23 | 300 |

**Exemple 14 : Simvastatin / γ-Cyclodextrine / DMF :**

Paramètres opératoires :

| Mélange | |
|---|---|
| Durée (h) | 2 |
| Pression (MPa) | 15 |
| Température (K) | 313 |
| Ratio molaire $CO_2$/solvant | 400 |
| Débit de solution (ml/min) | 1 |
| **Séchages** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

Protocole d'analyse des poudres : Test de dissolution :

*Conditions opératoires :*

Analyse UV à la longueur d'onde de 248 nm.

*Solution témoin :*

Préparer une solution étalon dans le lauryl sulfate de sodium à 1 % (m/v) dans $H_2O$. S'assurer de maintenir une absorbance <2.

*Réalisation de l'analyse :*

Préparer 50 ml d'une solution de Simvastatine dans de l'eau en introduisant une quantité de poudre équivalente à 50 mg de principe actif.

Laisser dissoudre sous agitation magnétique avec barreau aimanté, dans un bain marie à 37± 0,5°C.

Prélever 2ml de cette suspension après 2 heures d'agitation et filtrer sur filtre GELMAN GHP 0,45 μm.

Effectuer l'analyse UV en s'assurant que l'absorbance est inférieure à 2. Dans le cas contraire, effectuer une dilution.

*Résultat obtenu :*

Après 2 heures de dissolution, les concentrations (μg/ml) mesurées sont :

| Principe actif seul | Poudre après mélange | Poudre après procédé complet |
|---|---|---|
| < 1 | 13 | 212 |

**Exemple 15 : Terfénadine / β-cyclodextrine / DMSO :**

*Paramètres opératoires :*

| **Mélange** | |
|---|---|
| Durée (h) | 2 |
| Pression (MPa) | 15 |
| Température (K) | 313 |
| Ratio molaire $CO_2$/solvant | 400 |
| Débit de solution (ml/min) | 1 à 1,2 |
| **Séchages** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

*Protocole d'analyse des poudres : Test de dissolution :*

*Conditions opératoires :*

Analyse UV à la longueur d'onde de 259 nm.

*Solution témoin :*

Préparer une solution étalon dans le lauryl sulfate de sodium à 1 % (m/v) dans $H_2O$. S'assurer de maintenir une absorbance <2.

*Réalisation de l'analyse :*

Préparer 50 ml d'une solution de Terfénadine dans de l'eau en introduisant une quantité de poudre équivalente à 50 mg de principe actif.
Laisser dissoudre sous agitation magnétique avec barreau aimanté, dans un bain marie à 37± 0,5°C.
Prélever 2ml de cette suspension après 2 heures d'agitation et filtrer sur filtre
GELMAN GHP 0,45 μm.
Effectuer l'analyse UV en s'assurant que l'absorbance est inférieure à 2. Dans le cas contraire, effectuer une dilution.

*Résultat obtenu :*

Après 2 heures de dissolution, les concentrations (μg/ml) mesurées sont :

| Principe actif seul | Poudre après mélange | Poudre après procédé complet |
|---|---|---|
| < 1 | 290 | 990 |

**Exemple 16 : Terfénadine / β-cyclodextrine / DMF :**

Paramètres opératoires :

| | |
|---|---|
| **Mélange** | |
| Durée (h) | 2 |
| Pression (MPa) | 15 |
| Température (K) | 313 |
| Ratio molaire $CO_2$/solvant | 400 |
| Débit de solution (ml/min) | 1 à 1,2 |
| **Séchages** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

Protocole d'analyse des poudres : Test de dissolution :

*Conditions opératoires :*

Analyse UV à la longueur d'onde de 259 nm.

*Solution témoin* :

Préparer une solution étalon dans le lauryl sulfate de sodium à 1 % (m/v) dans $H_2O$.

S'assurer de maintenir une absorbance <2.

*Réalisation de l'analyse* :

Préparer 50 ml d'une solution de Terfénadine dans de l'eau en introduisant une quantité de poudre équivalente à 50 mg de principe actif.

Laisser dissoudre sous agitation magnétique avec barreau aimanté, dans un bain marie à 37± 0,5°C.

Prélever 2ml de cette suspension après 2 heures d'agitation et filtrer sur filtre GELMAN GHP 0,45 μm.

Effectuer l'analyse UV en s'assurant que l'absorbance est inférieure à 2. Dans le cas contraire, effectuer une dilution.

*Résultat obtenu* :

Après 2 heures de dissolution, les concentrations (μg/ml) mesurées sont :

| Principe actif seul | Poudre après procédé complet |
| --- | --- |
| < 1 | 323 |

**Exemple 17 : Terfénadine / Méthyl-β-cyclodextrine / Ethanol :**

Paramètres opératoires :

| Mélange | |
| --- | --- |
| Durée (h) | 2 |
| Pression (MPa) | 15 |
| Température (K) | 313 |
| Ratio molaire $CO_2$/solvant | 400 |
| Débit de solution (ml/min) | 1 à 1,2 |
| **Séchages** | |
| Durée (h) | 1 |
| Pression (MPa) | 15 |
| Température (K) | 313 |
| Débit de $CO_2$ (kg/h) | 15 |
| **Inclusion** | |
| Ajout d'eau (% masse totale) | 25 |
| Durée (h) | 16 |
| Pression (MPa) | 15 |
| Température (K) | 333 |

Protocole d'analyse des poudres : Test de dissolution :

*Conditions opératoires :*

Analyse UV à la longueur d'onde de 259 nm.

*Solution témoin :*

Préparer une solution étalon dans le lauryl sulfate de sodium à 1 % (m/v) dans $H_2O$. *S'assurer de maintenir une absorbance <2.*

*Réalisation de l'analyse :*

Préparer 50 ml d'une solution de Terfénadine dans de l'eau en introduisant une quantité de poudre équivalente à 50 mg de principe actif.

Laisser dissoudre sous agitation magnétique avec barreau aimanté, dans un bain marie à 37± 0,5°C.

Prélever 2ml de cette suspension après 2 heures d'agitation et filtrer sur filtre GELMAN GHP 0,45 μm.

Effectuer l'analyse UV en s'assurant que l'absorbance est inférieure à 2. Dans le cas contraire, effectuer une dilution.

*Résultat obtenu :*

Après 2 heures de dissolution, les concentrations (µg/ml) mesurées sont :

| Principe actif seul | Poudre après mélange | Poudre après procédé complet |
|---|---|---|
| < 1 | 420 | 552 |

Le tableau suivant rassemble les différents résultats de dissolution (µg/ml) obtenus pour toutes les molécules testées :

| | | | Dissolution à 2 heures | | |
|---|---|---|---|---|---|
| **MOLECULE** | **SOLVANT** | **CYCLO** | **Principe Actif seul** | **Poudre après co-cristallisation** | **Poudre après procédé complet** |
| F12511 | DMSO | β | 12 | 100 | 662 |
| Kétoprofène | DMSO | β | 333 | X | 923 |
| Oméprazole | DMSO | γ | 91 | X | 129 |
| | DMF | γ | 91 | X | 216 |
| Simvastatine | DMSO | γ | <1 | 23 | 300 |
| | DMF | γ | <1 | 13 | 212 |
| Terfénadine | DMSO | β | <1 | 290 | 990 |
| | DMF | β | <1 | X | 323 |
| | ETHANOL | Méthyl-β | <1 | 420 | 552 |
| NB : les cases indiquant X correspondent à des échantillons non prélevés. | | | | | |

Au vu de ces résultats, il est clair que le procédé qui permet d'obtenir la plus importante dissolution des principes actifs dans un milieu aqueux est celui combinant les étapes de mélange de principe actif et de support poreux, avantageusement de la cyclodextrine, de diffusion moléculaire et de séchage. Cette propriété est observée pour différents principes actifs, différents types de cyclodextrines, et différents solvants.

## Revendications

**1.** Procédé de préparation de composés d'interaction d'une substance active peu soluble dans un milieu aqueux avec un support poreux, **caractérisé en ce qu'**il comprend les étapes suivantes :

(a) Mélanger la substance active générée par fluide supercritique et la quantité déterminée de support poreux,
(b) Mettre en oeuvre une étape de diffusion moléculaire par mise en contact en mode statique d'un fluide supercritique avec le mélange obtenu à l'étape (a) pendant le temps nécessaire pour améliorer la dissolution dans un milieu aqueux du mélange obtenu à l'étape (a),
(c) Laver le composé d'interaction obtenue à l'étape (b) par un flux de fluide supercritique,
(c) Récupérer les particules du composé d'interaction ainsi formé.

**2.** Procédé selon la revendication 1, **caractérisé en ce que** le support poreux est générée par fluide supercritique et **en ce que** l'étape (a) comprend les étapes suivantes :

(a1) Mettre en solution la substance active et le support poreux dans un solvant organique, ledit solvant organique étant soluble dans le fluide supercritique,
(a2) Mettre en contact de façon continue la solution obtenue à l'étape (a1) avec ledit fluide supercritique, afin de désolvater de façon contrôlée la substance active et le support, et assurer leur coacervation,
(a3) Laver le complexe ainsi formé par extraction du solvant résiduel par le fluide supercritique, puis procéder à la séparation du solvant à l'état liquide et du fluide supercritique à l'état gazeux.

**3.** Procédé selon la revendication 1, **caractérisé en ce que** la substance active, avant son utilisation dans l'étape (a) est générée par le procédé comprenant les étapes suivantes :

(i) Mettre en solution la substance active dans un solvant organique, ledit solvant organique étant soluble dans le fluide supercritique,
(ii) Mettre en contact de façon continue la solution obtenue à l'étape (i) avec ledit fluide supercritique, afin de désolvater la substance active, et assurer sa coacervation,
(iii) Laver les particules de substance active ainsi formées par extraction du solvant résiduel par ledit fluide supercritique, puis procéder à la séparation du solvant à l'état liquide et du fluide supercritique à l'état gazeux,

et que le support poreux utilisé à l'étape (a) est sous forme solide.

**4.** Procédé selon la revendication 1, **caractérisé en ce que** la substance active, avant son utilisation dans l'étape (a) est générée par le procédé comprenant les étapes suivantes :

(i) Extraire la substance active par le fluide supercritique, éventuellement additionné d'un co-solvant,
(ii) Vaporiser le mélange supercritique afin de désolvater la substance active, et assurer sa coacervation,
(iii) Laver les particules de substance active ainsi formées par le fluide supercritique, puis éventuellement procéder à la séparation du co-solvant à l'état liquide et du fluide supercritique à l'état gazeux,

et **en ce que** le support poreux utilisé à l'étape (a) est sous forme solide.

**5.** Procédé selon la revendication 1, **caractérisé en ce que** l'étape (a) comprend les étapes suivantes :

(a1) Mettre en solution la substance active dans un solvant organique, ledit solvant organique étant soluble dans le fluide supercritique,
(a2) Mettre en contact de façon continue la solution ainsi obtenue avec le fluide supercritique, afin de désolvater la substance active, et assurer sa coacervation sur le support poreux préalablement placé dans le réacteur,
(a3) Laver le complexe ainsi formé par extraction du solvant résiduel par le fluide supercritique, puis procéder à la séparation du solvant à l'état liquide et du fluide supercritique à l'état gazeux.

**6.** Procédé selon la revendication 1, **caractérisé en ce que** l'étape (a) comprend les étapes suivantes :

(a1) Extraire la substance active par un fluide supercritique, éventuellement additionné d'un co-solvant,
(a2) Vaporiser le mélange supercritique afin de désolvater la substance active, et assurer sa coacervation sur le support poreux préalablement placé dans le réacteur,
(a3) Laver le complexe ainsi formé par le fluide supercritique, puis éventuellement procéder à la séparation du co-solvant à l'état liquide et du fluide supercritique à l'état gazeux.

**7.** Procédé selon l'une quelconque des revendications 2 à 6, **caractérisé en ce que** le solvant organique ou le co-solvant est choisit dans le groupe constitué par les alcools, les cétones, l'acide acétique, l'acétate d'éthyle, le dichlorométhane, l'acétonitrile, le diméthylformamide, le diméthylsulfoxide et leur mélange.

**8.** Procédé selon l'une quelconque des revendications précédentes, **caractérisé en ce que** le fluide supercritique est du CO2.

**9.** Procédé selon l'une quelconque des revendications précédentes, **caractérisé en ce que** la substance active est choisi dans le groupe constitué par les dérivés d'anilides, en particulier le (S)-2',3',5'-triméthyl-4'-hydroxy-α-dodécylthiophényl acétanilide, les dérivés d'épipodophyllotoxine, en particulier le 4'-déméthyl-4'-désoxy-4'-phosphate-4-0-(2,3-bis-(2,3,4,5,6-pentafluorophénoxyacétyl)-4,6-éthylidène-β-D-glucosyl)-épipodophyllotoxine, le piroxicam, l'acide valérique, l'acide octanoïque, l'acide laurique et l'acide stéarique.

**10.** Procédé selon l'une quelconque des revendications précédentes, **caractérisé en ce que** le support poreux est choisi dans le groupe constitué par les cyclodextrines et leur mélange.

**11.** Procédé selon l'une quelconque des revendications précédentes, **caractérisé en ce que** l'étape (b) de diffusion moléculaire est réalisé sous agitation.

**12.** Procédé selon l'une quelconque des revendications précédentes, **caractérisé en ce que** l'étape (b) de diffusion moléculaire est réalisée en présence d'un agent de diffusion.

**13.** Procédé selon la revendication 12, **caractérisé en ce que** l'agent de diffusion est choisi dans le groupe constitué par l'alcool, l'eau avec ou sans agent surfactant et leur mélanges.

**14.** Procédé selon l'une quelconque des revendications précédentes, **caractérisé en ce que** la pression du fluide supercritique est comprise entre 10 MPa et 40 MPa et la température entre 0 et 120°C.

**15.** Procédé selon l'une quelconque des revendications précédentes, **caractérisé en ce que** chacune des étapes du procédé est mise en oeuvre dans un réacteur fermé, en particulier un autoclave.

**16.** Procédé selon l'une quelconque des revendications précédentes, **caractérisé en ce qu'**il est réalisé en continu.

**17.** Composé d'interaction d'une substance active peu soluble dans un milieu aqueux dans un support poreux **caractérisé en ce qu'**il est susceptible d'être obtenu par le procédé selon l'une quelconque des revendications 1 à 16.

**18.** Composé selon la revendication 17 **caractérisé en ce que** la substance active ainsi complexée présente une solubilité dans une solution aqueuse de Laurylsulfate de sodium à 5 % supérieure à environ 600 μg/ml

## Claims

**1.** Method for preparing compounds of interaction of an active substance of low solubility in an aqueous medium with a porous support, **characterized in that** it comprises the following steps:

(a) mixing the active substance generated by supercritical fluid and the defined amount of porous support,
(b) implementing a step of molecular diffusion by contacting in static mode a supercritical fluid with the mixture obtained in step (a) for the time required to improve the dissolution in an aqueous medium of the mixture obtained in step (a),
(c) washing the interaction compound obtained in step (b) with a flow of supercritical fluid,
(d) recovering the particles of the interaction compound thus formed.

**2.** Method according to Claim 1, **characterized in that** the porous support is generated by supercritical fluid and **in that** step (a) comprises the following steps:

(a1) dissolving the active substance and the porous support in an organic solvent, said organic solvent being soluble in the supercritical fluid,
(a2) continuously contacting the solution obtained in step (a1) with said supercritical fluid, so as to effect controlled desolvation of the active substance and the support, and to ensure their coacervation,
(a3) washing the complex thus formed by extracting the residual solvent using the supercritical fluid, then separating the solvent in the liquid state and the supercritical fluid in the gaseous state.

**3.** Method according to Claim 1, **characterized in that** the active substance, before being used in step (a), is generated by the process comprising the following steps:

(i) dissolving the active substance in an organic solvent, said organic solvent being soluble in the supercritical fluid,
(ii) continuously contacting the solution obtained in step (i) with said supercritical fluid, so as to effect desolvation of active substance, and to ensure its coacervation,
(iii) washing the particles of active substance thus formed by extracting the residual solvent using said supercritical fluid, then separating the solvent in the liquid state and the supercritical fluid in the gaseous state,

the porous support used in step (a) being in solid form.

**4.** Method according to Claim 1, **characterized in that** the active substance, before being used in step (a), is generated by the process comprising the following steps:

(i) extracting the active substance with the supercritical fluid, optionally admixed with a cosolvent,
(ii) vaporizing the supercritical mixture so as to effect desolvation of the active substance, and to ensure its coacervation,
(iii) washing the particles of active substance thus formed with the supercritical fluid, then optionally separating the cosolvent in the liquid state and the supercritical fluid in the gaseous state, and **in that** the porous support used in step (a) is in solid form.

**5.** Method according to Claim 1, **characterized in that** the step (a) comprises the following steps:

(a1) dissolving the active substance in an organic solvent, said organic solvent being soluble in the supercritical fluid,
(a2) continuously contacting the solution thus obtained with the supercritical fluid, so as to effect desolvation of the active substance, and to ensure its coacervation on the porous support placed in the reactor beforehand,
(a3) washing the complex thus formed by extracting the residual solvent using the supercritical fluid, then separating the solvent in the liquid state and the supercritical fluid in the gaseous state.

**6.** Method according to Claim 1, **characterized in that** step (a) comprises the following steps:

(a1) extracting the active substance with a supercritical fluid, optionally admixed with a cosolvent,
(a2) vaporizing the supercritical mixture so as to effect desolvation of the active substance, and to ensure its coacervation on the porous support placed in the reactor beforehand,
(a3) washing the complex thus formed with the supercritical fluid, then optionally separating the cosolvent in the liquid state and the supercritical fluid in the gaseous state.

**7.** Method according to any one of Claims 2 to 6, **characterized in that** the organic solvent or cosolvent is selected from the group consisting of alcohols, ketones, acetic acid, ethyl acetate, dichloromethane, acetonitrile, dimethylformamide, dimethyl sulfoxide, and a mixture thereof.

**8.** Method according to any one of the preceding claims, **characterized in that** the supercritical fluid is $CO_2$.

**9.** Method according to any one of the preceding claims, **characterized in that** the active substance is selected from the group consisting of anilide derivatives, in particular (S)-2',3',5'-trimethyl-4'-hydroxy-α-dodecylthiophenylacetanilide, epipodophyllotoxin derivatives, in particular 4'-dimethyl-4'-deoxy-4'-phosphate-4-O-(2,3-bis-(2,3,4,5,6-pentafluorophenoxyacetyl)-4,6-ethylidene-β-D-glucosyl) epipdodophyllotoxin, piroxicam, valeric acid, octanoic acid, lauric acid, and stearic acid.

**10.** Method according to any one of the preceding claims, **characterized in that** the porous support is selected from the group consisting of cyclodextrins and a mixture thereof.

**11.** Method according to any one of the preceding claims, **characterized in that** step (b) of molecular diffusion is carried out with stirring.

**12.** Method according to any one of the preceding claims, **characterized in that** step (b) of molecular diffusion is carried out in the presence of a diffusion agent.

**13.** Method according to Claim 12, **characterized in that** the diffusion agent is selected from the group consisting of alcohol, water with or without surfactant, and mixtures thereof.

**14.** Method according to any one of the preceding claims, **characterized in that** the pressure of the supercritical fluid is between 10 MPa and 40 MPa and the temperature is between 0 and 120°C.

**15.** Method according to any one of the preceding claims, **characterized in that** each of the steps of Method is implemented in a closed reactor, in particular an autoclave.

**16.** Method according to any one of the preceding claims, **characterized in that** it is carried out continuously.

**17.** Compound of interaction of an active substance of low solubility in an aqueous medium in a porous support, **characterized in that** it is obtainable by the method according to any one of Claims 1 to 16.

**18.** Compound according to Claim 17, **characterized in that** the active substance thus complexed has a solubility in 5% aqueous sodium lauryl sulfate solution of greater than approximately 600 μg/ml.

**Patentansprüche**

**1.** Verfahren zur Herstellung von Wechselwirkungsverbindungen eines in wässrigem Medium wenig löslichen Wirkstoffs mit einem porösen Träger, **dadurch gekennzeichnet, dass** es die folgenden Schritte umfasst:

(a) Mischen des Wirkstoffs, der durch superkritisches Fluid erzeugt wurde, und der festgelegten Menge an porösem Träger,
(b) Durchführen eines Schrittes der molekularen Diffusion durch statisches In-Kontakt-Bringen eines superkritischen Fluids mit der im Schritt (a) erhaltenen Mischung über die erforderliche Zeit, um die Lösung der im Schritt (a) erhaltenen Mischung in wässrigem Milieu zu verbessern,
(c) Waschen der im Schritt (b) erhaltenen Wechselwirkungsverbindung mit einem Strom von superkritischem Fluid,
(c) Gewinnen der Teilchen der so gebildeten Wechselwirkungsverbindung.

**2.** Verfahren nach Anspruch 1, **dadurch gekennzeichnet, dass** der poröse Träger durch superkritisches Fluid erzeugt wird und dass der Schritt (a) die folgenden Schritte umfasst:

(a1) Lösen des Wirkstoffs und des porösen Trägers in einem organischen Lösungsmittel, wobei das organische Lösungsmittel in dem superkritischen Fluid löslich ist,
(a2) kontinuierliches In-Kontakt-Bringen der im Schritt (a1) erhaltenen Lösung mit dem superkritischen Fluid, um auf kontrollierte Weise den Wirkstoff und den Träger zu desolvatisieren und deren Koazervation sicherzustellen,
(a3) Waschen des so gebildeten Komplexes durch Extraktion des rückständigen Lösungsmittels mit superkritischem Fluid, dann Vornehmen der Trennung des Lösungsmittels in flüssigem Zustand und des superkritischen Fluids in gasförmigem Zustand.

**3.** Verfahren nach Anspruch 1, **dadurch gekennzeichnet, dass** der Wirkstoff vor seiner Verwendung im Schritt (a) durch das Verfahren erzeugt wird, das die folgenden Schritte umfasst:

(i) Lösen des Wirkstoffs in einem organischen Lösungsmittel, wobei das organische Lösungsmittel in dem superkritischen Fluid löslich ist,
(ii) kontinuierliches In-Kontakt-Bringen der im Schritt (i) erhaltenen Lösung mit dem superkritischen Fluid, um den Wirkstoff zu desolvatisieren und seine Koazervation sicherzustellen,
(iii) Waschen der so gebildeten Wirkstoffteilchen durch Extraktion des rückständigen Lösungsmittels durch das superkritische Fluid, dann Vornehmen der Trennung des Lösungsmittels in flüssigem Zustand und des superkritischen Fluids in gasförmigem Zustand,

und dass der im Schritt (a) verwendete poröse Träger in fester Form vorliegt.

**4.** Verfahren nach Anspruch 1, **dadurch gekennzeichnet, dass** der Wirkstoff vor seiner Verwendung im Schritt (a) durch das Verfahren erzeugt wird, das die folgenden Schritte umfasst:

(i) Extrahieren des Wirkstoffs mit superkritischem Fluid, das gegebenenfalls mit einem Cosolvens versetzt ist,
(ii) Verdampfen der superkritischen Mischung, um den Wirkstoff zu desolvatisieren und seine Koazervation sicherzustellen,
(iii) Waschen der so gebildeten Wirkstoffteilchen mit dem superkritischen Fluid, dann gegebenenfalls Vornehmen der Trennung des Cosolvens in flüssigem Zustand und des superkritischen Fluids in gasförmigem Zustand,

und **dadurch**, dass der im Schritt (a) verwendete poröse Träger in fester Form vorliegt.

**5.** Verfahren nach Anspruch 1, **dadurch gekennzeichnet, dass** der Schritt (a) die folgenden Schritte umfasst:

(a1) Lösen des Wirkstoffs in einem organischen Lösungsmittel, wobei das organische Lösungsmittel in dem superkritischen Fluid löslich ist,

(a2) Kontinuierliches In-Kontakt-Bringen der so erhaltenen Lösung mit dem superkritischen Fluid, um den Wirkstoff zu desolvatisieren und seine Koazervation über dem festen Träger, der zuvor in den Reaktor gegeben wurde, sicherzustellen,
(a3) Waschen des so gebildeten Komplexes durch Extraktion des rückständigen Lösungsmittels mit superkritischem Fluid, dann Vornehmen der Trennung des Lösungsmittels in flüssigem Zustand und des superkritischen Fluids in gasförmigem Zustand.

**6.** Verfahren nach Anspruch 1, **dadurch gekennzeichnet, dass** der Schritt (a) die folgenden Schritte umfasst:

(a1) Extrahieren des Wirkstoffs mit einem superkritischen Fluid, das gegebenenfalls mit einem Cosolvens versetzt ist,
(a2) Verdampfen der superkritischen Mischung, um den Wirkstoff zu desolvatisieren und seine Koazervation über dem festen Träger, der zuvor in den Reaktor gegeben wurde, sicherzustellen,
(a3) Waschen des so gebildeten Komplexes mit superkritischem Fluid, dann gegebenenfalls Vornehmen der Trennung des Lösungsmittels in flüssigem Zustand und des superkritischen Fluids in gasförmigem Zustand.

**7.** Verfahren nach irgendeinem der Ansprüche 2 bis 6, **dadurch gekennzeichnet, dass** das organische Lösungsmittel oder das Cosolvens ausgewählt ist aus der Gruppe bestehend aus Alkoholen, Ketonen, Essigsäure, Ethylacetat, Dichlormethan, Acetonitril, Dimethylformamid, Dimethylsulfoxid und deren Mischungen.

**8.** Verfahren nach irgendeinem der vorangehenden Ansprüche, **dadurch gekennzeichnet, dass** das superkritische Fluid $CO_2$ ist.

**9.** Verfahren nach irgendeinem der vorangehenden Ansprüche, **dadurch gekennzeichnet, dass** der Wirkstoff ausgewählt ist aus der Gruppe bestehend aus Anilid-Derivaten, insbesondere (S)-2',3',5'-Trimethyl-4'-hydroxy-α-dodecylthiophenylacetanilid, Epipodophyllotoxin-Derivaten, insbesondere 4'-Demethyl-4'-desoxy-4'-phosphat-4-0-(2,3-bis(2,3,4,5,6-pentafluorphenoxyacetyl)-4,6-ethyliden-β-D-glucosyl)epipodophyllotoxin, Piroxicam, Valeriansäure, Octansäure, Laurinsäure und Stearinsäure.

**10.** Verfahren nach irgendeinem der vorangehenden Ansprüche, **dadurch gekennzeichnet, dass** der poröse Träger ausgewählt ist aus der Gruppe bestehend aus Cyclodextrinen und deren Mischungen.

**11.** Verfahren nach irgendeinem der vorangehenden Ansprüche, **dadurch gekennzeichnet, dass** der Schritt (b) der molekularen Diffusion unter Rühren durchgeführt wird.

**12.** Verfahren nach irgendeinem der vorangehenden Ansprüche, **dadurch gekennzeichnet, dass** der Schritt (b) der molekularen Diffusion in Anwesenheit eines Diffusionsmittels durchgeführt wird.

**13.** Verfahren nach Anspruch 12, **dadurch gekennzeichnet, dass** das Diffusionsmittel ausgewählt ist aus der Gruppe bestehend aus Alkohol, Wasser mit oder ohne Tensid und deren Mischungen.

**14.** Verfahren nach irgendeinem der vorangehenden Ansprüche, **dadurch gekennzeichnet, dass** der Druck des superkritischen Fluids zwischen 10 MPa und 40 MPa einschließlich und die Temperatur zwischen 0 und 120°C einschließlich liegt.

**15.** Verfahren nach irgendeinem der vorangehenden Ansprüche, **dadurch gekennzeichnet, dass** jeder der Schritte des Verfahrens in einem geschlossenen Reaktor, insbesondere einem Autoklaven, durchgeführt wird.

**16.** Verfahren nach irgendeinem der vorangehenden Ansprüche, **dadurch gekennzeichnet, dass** es kontinuierlich durchgeführt wird.

**17.** Wechselwirkungsverbindung eines in wässrigem Medium wenig löslichen Wirkstoffs in einem porösen Träger, **dadurch gekennzeichnet, dass** sie durch das Verfahren nach irgendeinem der Ansprüche 1 bis 16 erhältlich ist.

**18.** Verbindung nach Anspruch 17, **dadurch gekennzeichnet, dass** der so komplexierte Wirkstoff in einer 5 %-igen wässrigen Lösung von Natriumlaurylsulfat eine Löslichkeit von mehr als etwa 600 μg/ml aufweist.

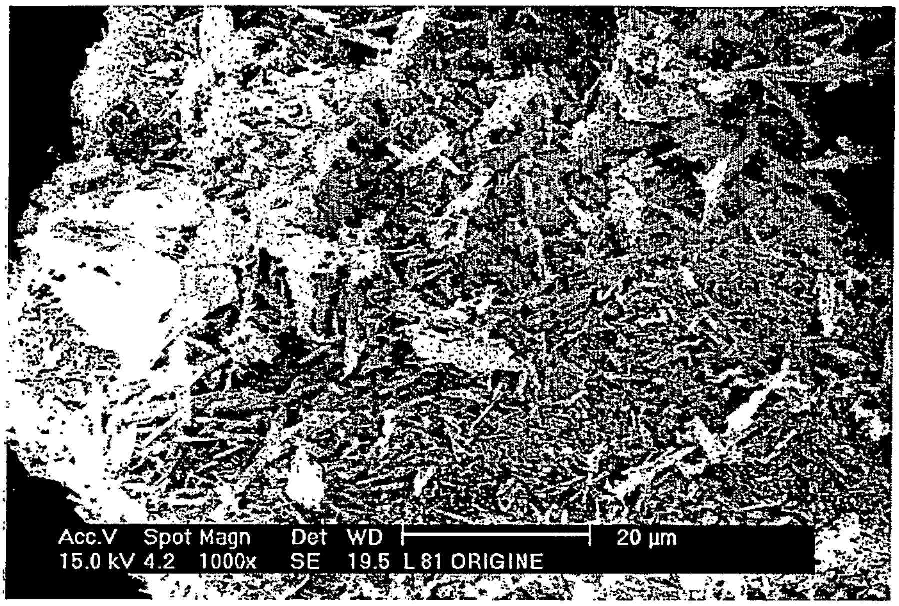
Acc.V Spot Magn Det WD 20 µm
15.0 kV 4.2 1000x SE 19.5 L81 ORIGINE

FIG.1

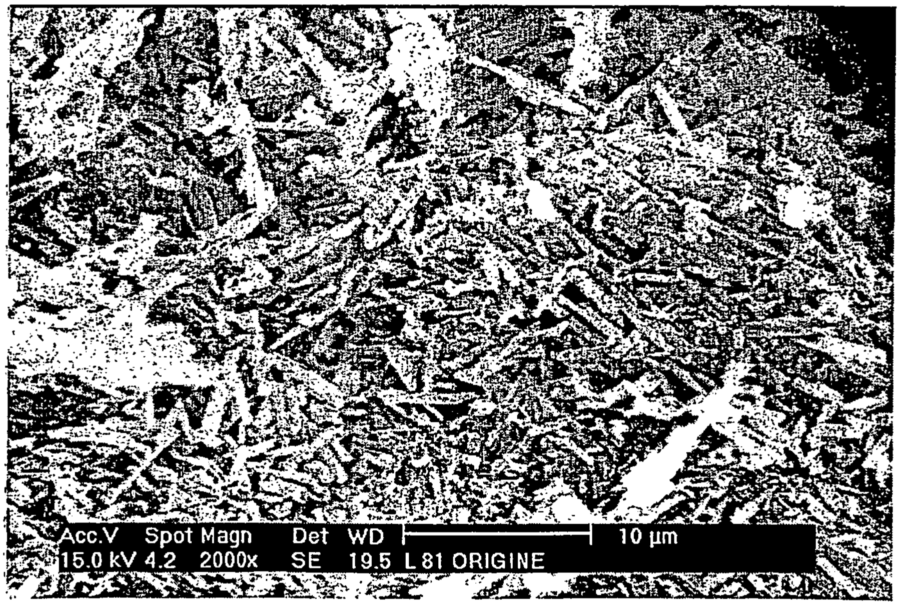
Acc.V Spot Magn Det WD 10 µm
15.0 kV 4.2 2000x SE 19.5 L81 ORIGINE

FIG.2

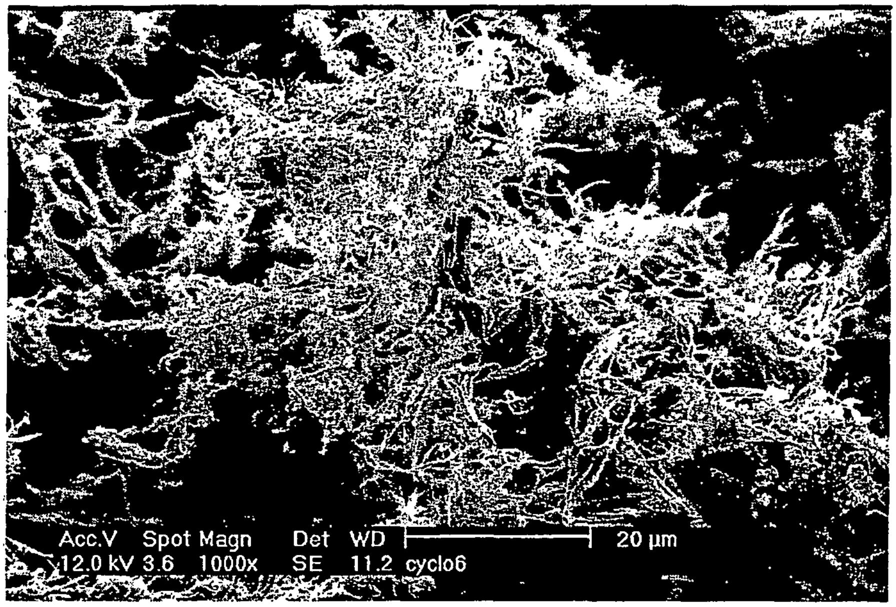
Acc.V Spot Magn Det WD 20 µm
12.0 kV 3.6 1000x SE 11.2 cyclo6

FIG.3

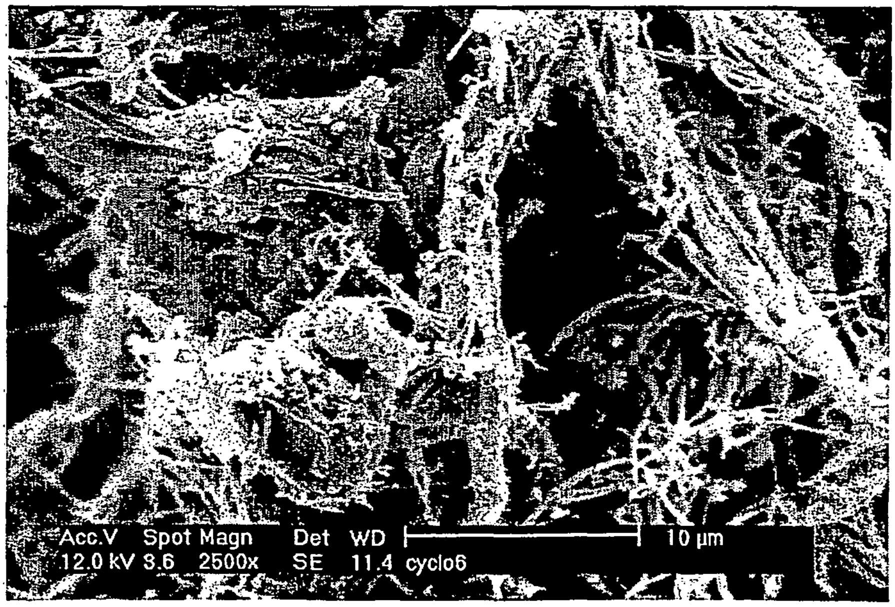
Acc.V Spot Magn Det WD 10 µm
12.0 kV 3.6 2500x SE 11.4 cyclo6

FIG.4

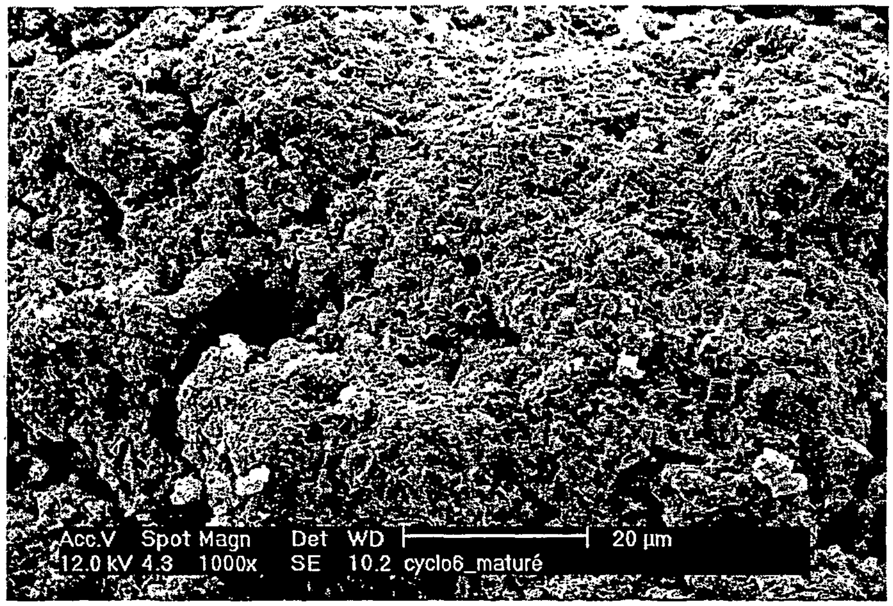
Acc.V Spot Magn Det WD 20 µm
12.0 kV 4.3 1000x SE 10.2 cyclo6_maturé

FIG.5

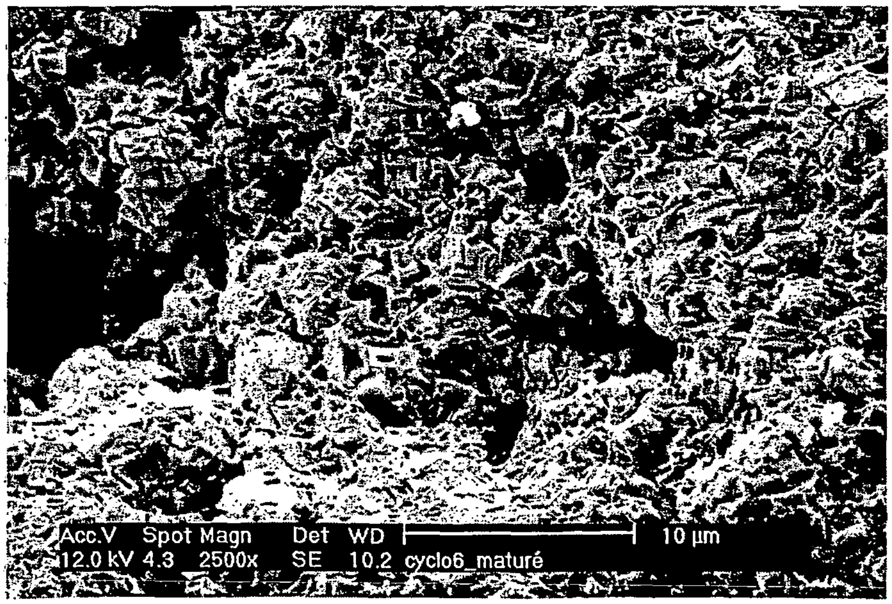
Acc.V Spot Magn Det WD 10 µm
12.0 kV 4.3 2500x SE 10.2 cyclo6_maturé

FIG.6

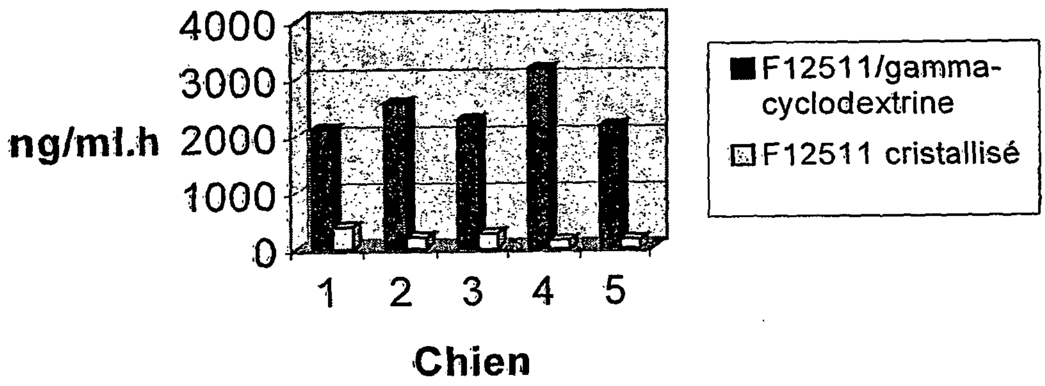
4000
3000
2000
1000
0
ng/ml.h
1
2
3
4
5
Chien
F12511/gamma-cyclodextrine
F12511 cristallisé

FIG.7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9813136 A
- EP 706821 A
- WO 9815348 A
- FR 2798863
- WO 9731691 A
- WO 9925322 A
- US 5043280 A
- FR 2815540
- FR 2741619
- FR 2725990


### Littérature non-brevet citée dans la description


- Drugs encapsulation using a compressed gas antisolvent technique. **BERTUCCO et al.** Proceedings of the 4th Italian Conference on Supercritical Fluids and their Applications. 1997, 327-334
- **CHOU et al.** GAS crystallization of polymer-pharmaceutical composite particles. *Proceedings of the 4'th International Symposium on Supercritical Fluids,* 1997, 55-57
- **KIM J.-H. et al.** Microencapsulation of Naproxen using Rapid Expansion of Supercritical Solutions. *Biotechnol. Prog.,* 1996, vol. 12, 650-661
- **SZE TU et al.** Applications of dense gases in pharmaceutical processing. *Proceedings of the 5th Meeting on Supercritical Fluids,* 1998, vol. 1, 263-269
- **WEBER et al.** Coprecipitation with compressed antisolvents for the manufacture of microcomposites. *Proceedings of the 5th Meeting on Supercritical Fluids,* 1998, vol. 1, 243-248
- **BLEICH ; MÜLLER.** Production of drug loaded by the use of supercritical gases with the Aerosol Solvent Extraction System (ASES) process. *J. Microencapsulation,* 1996, vol. 13, 131-139
- Applications of supercritical fluids in controlled release of drugs. **TOM et al.** Supercritical Fluids Engineering Science ACS Symp. Ser. 514. American Chemical Society, 1992
- **VAN HEES et al.** Application of supercritical carbon dioxide for the preparation of a Piroxicam-β-cyclodextrin inclusion compound. *Pharmaceutical Research,* 1999, vol. 16 (12
- **KAMIHIRA M. et al.** Formation of inclusion complexes between cyclodextrins and aromatic compounds under pressurized carbon dioxide. *J. of Fermentation and Bioengineering,* 1990, vol. 69 (6), 350-353
- **SZE TU L. et al.** Application of dense gazes in pharmaceutical processing. *Proceedings of 5th meeting on supercritical fluids,* Mars 1998